(19) Europäisches Patentamt
European Patent Office
Office européen des brevets

(11) EP 2 216 051 A1

(12) # EUROPEAN PATENT APPLICATION

published in accordance with Art. 153(4) EPC

(43) Date of publication:
**11.08.2010 Bulletin 2010/32**

(21) Application number: **08844437.7**

(22) Date of filing: **28.10.2008**

(51) Int Cl.:
***A61K 51/00*** *(2006.01)* ***C07D 471/04*** *(2006.01)*

(86) International application number:
**PCT/JP2008/069501**

(87) International publication number:
**WO 2009/057575 (07.05.2009 Gazette 2009/19)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.10.2007 JP 2007282361**

(71) Applicant: **Nihon Medi-Physics Co., Ltd.**
**Tokyo 136-0075 (JP)**

(72) Inventors:
- **TANIFUJI, Shigeyuki**
**Sodegaura-shi**
**Chiba 299-0266 (JP)**
- **NAKAMURA, Daisaku**
**Sodegaura-shi**
**Chiba 299-0266 (JP)**
- **TAKASAKI, Shinya**
**Sodegaura-shi**
**Chiba 299-0266 (JP)**
- **OKUMURA, Yuki**
**Sodegaura-shi**
**Chiba 299-0266 (JP)**

(74) Representative: **Wilhelms · Kilian & Partner**
**Patentanwälte**
**Eduard-Schmid-Straße 2**
**81541 München (DE)**

(54) **USE OF NOVEL COMPOUND HAVING AFFINITY FOR AMYLOID, AND PROCESS FOR PRODUCTION OF THE SAME**

(57) The invention provides a reagent for detecting amyloid in a biological tissue which can detect amyloid *in vitro* and *in vivo* with high sensitivity using a compound which has affinity with amyloid and is suppressed in toxicity such as mutagenicity.

The reagent for detecting amyloid deposited in a biological tissue comprises the compound represented by the following formula (1) or a salt thereof:

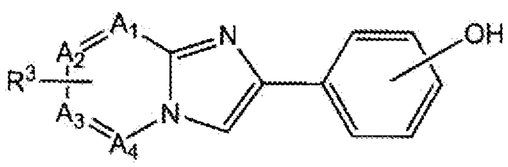

(1)

wherein $A^1$, $A^2$, $A^3$ and $A^4$ independently represent a carbon or a nitrogen, and$R^3$ is a group represented by the following formula:

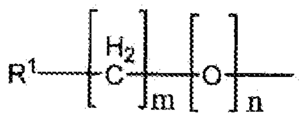

EP 2 216 051 A1

wherein $R^1$ is a radioactive halogen substituent; m is an integer of 0 to 4; and n is an integer of 0 or 1, provided that at least one of $A^1$, $A^3$, $A^3$ and $A^4$ represents a carbon, and $R^5$ binds to a carbon represented by $A^1$, $A^2$, $A^3$ or $A^4$.

**Description**

TECHNICAL FIELD

The present invention relates to use and process for production of a novel compound having affinity for amyloid, and especially relates to a reagent for detecting amyloid in biological tissues, which is useful for detection of amyloid at lesion sites in diagnosis of systemic amyloidosis diseases and other diseases with amyloid accumulation.

BACKGORUND ART

Diseases with the onset of deposition of a fibrous protein called amyloid in various organs or tissues in bodies are generally referred to as amyloidosis. A feature common to amyloidosis is that the fibrous protein called amyloid which is enriched with the β-sheet structure is deposited at various organs systemically or at sites topically so that functional abnormalities are triggered in the organs or tissues. Amyloid generally refers to protein aggregates formed by aggregation of various amyloid precursor proteins such as amyloid-β, mutant transthyretin and β2-microglobulin in a living body. The amyloid has a characteristic structure enriched with β-sheet even if it is formed of any of the amyloid precursor proteins. Thus, compounds such as Congo-red and Thioflavin T capable of binding to β-sheet are characteristic in that they have affinity with amyloid.

Amyloidosis is classified into a systemic amyloidosis and a localized amyloidosis depending upon amyloid deposition patterns.

The systemic amyloidosis is a disease in which amyloid deposition occurs at various parts of the whole body. Examples of the systemic amyloidosis include familial amyloidosis in which amyloid produced in the lever is deposited in organs throughout the whole body so as to cause disorder, senile TTR amyloidosis in which amyloid is deposited in heart and a large joint such as hand joint, dialysis amyloidosis occurring at sites such as bones and joints of long-term dialysis patients, reactive AA amyloidosis (secondary amyloidosis) which is caused by deposition of amyloid derived from serum amyloid A which is an acute phase protein produced following a chronic inflammatory disease such as chronic rheumatism, and immunocytic amyloidosis in which amyloid derived from immunoglobulin is deposited in various organs throughout the whole body.

The localized amyloidosis is a disease in which amyloid deposition occurs only at some organs. Examples of the localized amyloidosis include brain amyloidosis such as Alzheimer's disease in which amyloid is deposited in brain, cerebrovascular amyloidosis and Creutzfeldt Jakob disease, endocrine amyloidosis in which amyloid is deposited in pancreatic inslet accompanied by type II diabetes and insulinoma or deposited in heart atrium, cutaneous amyloidosis in which amyloid is deposited in skin, and localized nodular amyloidosis in which nodular amyloid is deposited in skin and lungs.

Diagnosis of amyloidosis is, in case of the systemic amyloidosis, at first conducted by collecting a tissue from a region where biopsy is available such as skin, kidney and gastrointestinal tracts, and staining it with Congo-red or Thioflavin T. Congo-red is a fluorescent compound high in affinity with β-sheet structure of amyloid, and since Congo-red shows double refraction under polarization microscope due to its orientation, it can selectively stain amyloid deposition in tissues. Similarly, Thioflavin T is also a fluorescent compound having affinity with amyloid, and used similarly to Congo-red. After the tissue staining is found to be positive, definite diagnosis is conducted by means of immunostaining with an antibody or the like in combination therewith. However, positive diagnosis is often difficult by staining with Congo-red and Thioflavin T even under polarization microscope.

On the other hand, imaging diagnosis of the systemic amyloidosis has been considered with recent wide spread of image diagnosis devices such as PET, SPECT and MRI.

However, when Congo-red and Thioflavin T are labeled and used as probes for imaging diagnosis, they are problematic in that binding specificity to amyloid is inferior so that good detection sensitivity cannot be obtained.

Further, since Congo-red has carcinogenicity, it cannot be used for diagnosis of human body.

Thus, bis-(3-hydroxycarbonyl-4-hydroxy)styrylbenzene (BSB) as a Congo-red derivative and derivatives thereof have been proposed as a fluorescent reagent for detecting amyloid which is high in affinity and detection sensitivity for systemic amyloid and can be used for *in vivo* detection (non-Patent Document 14, Patent Document 7). It has been reported that BSB is high in affinity for amyloid caused by brain amyloidosis and systemic amyloidosis, and has no benzizine structure in its structure, and thus it has little carcinogenic problem and can be radioactive-labeled for use as a probe for imaging diagnosis.

On the other hand, for Alzheimer's disease (hereinafter, referred to as AD) which is a typical brain amyloidosis, attempts have already been made to detect AD *in vivo* using a compound having high affinity with amyloid as a marker since it is impossible to collect a biopsy.

Many of such probes for imaging diagnoses of cerebral amyloid are hydrophobic low-molecular weight compounds that are high in affinity with amyloid and high in cerebral transferability and are labeled with various radioactive species such as $^{11}C$, $^{18}F$ and $^{123}I$. For example, reports tell $^{11}C$ or radioactive halogen labeled forms of compounds including various

thioflavin derivatives such as 6-iodo-2-[4'-(N,N-dimethylamino)phenyl]benzothiazole (hereinafter referred to as TZDM) and 6-hydroxy-2-[4'-(N-methylamino)phenyl]benzothiazole (hereinafter referred to as 6-OH-BTA-1) (Patent Document 1, Non-Patent Document 3); stilbene compounds such as (E)-4-methylamino-4'-hydroxystilbene (hereinafter referred to as SB-13) and (E)-4-dimethylamino-4'-iodostilbene (hereinafter referred to as m-I-SB) (Patent Document 2, Non-Patent Document 4, Non-Patent Document 5); benzoxazole derivatives such as 6-iodo-2-[4'-(N,N-dimethylamino)phenyl]benzoxazole (hereinafter referred to as IBOX) and 6-[2-(fluoro)ethoxy]-2-[2-(2-dimethylaminothiazol-5-yl)ethenyl]benzoxazole (Non-Patent Document 6, Non-Patent Document 7), DDNP derivatives such as 2-(1-{6-[(2-fluoroethyl) (methyl) amino]-2-naphthyl}ethylidene)malononitrile (hereinafter referred to as FDDNP) (Patent Document 4, Non-Patent Document 8); and imidazopyridine derivatives such as 6-iodo-2-[4'-(N,N-dimethylamino)phenyl]imidazo[1,2-a]pyridine (hereinafter referred to as IMPY) (Patent Document 3, Non-Patent Document 9). Further, some of these probes for imaging diagnosis have been studied on human imaging and have been reported to show a significant radioactivity accumulation in AD patient's brain compared with normal persons (Non-Patent Document 10, Non-Patent Document 11, Non-Patent Document 12, and Non-Patent Document 13).

International Publication No. WO2007/002540 pamphlet discloses a series of compounds with a group having affinity with amyloid, to which a radioisotope labeling site is attached via ethylene glycol or polyethylene glycol (Parent Document 5).

International Publication No. WO2007/063946 pamphlet discloses a series of compounds to which a five-membered aromatic heterocyclic group is attached in order to prevent them from being metabolized in brain (Patent Document 6).

[Patent Document 1] JP-T-2004-506723

[Patent Document 2] JP-T-2005-504055

[Patent Document 3] JP-T-2005-512945

[Patent Document 4] JP-T-2002-523383

[Patent Document 5] International Publication No. WO2007/002540 pamphlet

[Patent Document 6] International Publication No. WO2007/063946 pamphlet

[Patent Document 7] International Publication No. WO2005/016888 pamphlet

[Non-Patent Document 1] J. A. Hardy & G. A. Higgins, "Alzheimer's Disease: The Amyloid Cascade Hypothesis.", Science, 1992, 256, p.184-185

[Non-Patent Document 2] G. McKhann et al., "Clinical diagnosis of Alzheimer's disease: Report of the NINCDS-ADRDA Work Group under the auspices of Department of Health and Human Services Task Force on Alzheimer's Disease.", Neurology, 1984, 34, p.939-944

[Non-Patent Document 3] Z.-P. Zhuang et al., "Radioiodinated Styrylbenzenes and Thioflavins as Probes for Amyloid Aggregate.", J. Med. Chem., 2001, 44, p.1905-1914

[Non-Patent Document 4] Masahiro Ono et al., "11C-labeled stilbene derivatives as Aβ-aggregate-specific PET imaging agents for Alzheimer's disease.", Nuclear Medicine and Biology, 2003, 30, p.565-571

[Non-Patent Document 5] H. F. Kung et al., "Novel Stilbenes as Probes for amyloid plaques.", J. American Chemical Society, 2001, 123, p.12740-12741

[Non-Patent Document 6] Zhi-Ping Zhuang et al., "IBOX(2-(4'-dimethylaminophenyl)-6-iodobensoxazole): a ligand for imaging amyloid plaques in the brain.", Nuclear Medicine and Biology, 2001, 28, p.887-894

[Non-Patent Document 7] Furumoto Y et al., "[11C]BF-227: A New 11C-Labeled 2-Ethenylbenzoxazole Derivative for Amyloid-β Plaques Imaging.", European Journal of Nuclear Medicine and Molecular Imaging, 2005, 32, Sup.1, P759

[Non-Patent Document 8] Eric D. Agdeppa et al., "2-Dialkylamino-6-Acylmalononitrile Substituted Naphthalenes (DDNP Analogs): Novel Diagnostic and Therapeutic Tools in Alzheimer's Disease.", Molecular Imaging and Biology, 2003, 5, p.404-417

[Non-Patent Document 9] Zhi-Ping Zhuang et al., "Structure-Activity Relationship of Imidazo[1,2-a]pyridines as Ligands for Detecting β-Amyloid Plaques in the Brain.", J. Med. Chem, 2003, 46, p.237-243 [Non-Patent Document 10] W. E. Klunk et al., "Imaging brain amyloid in Alzheimer's disease with Pittsburgh Compound-B.", Ann. Neurol., 2004, 55, p.306-319

[Non-Patent Document 11] Nicolaas P. L. G. Verhoeff et al., "In-Vivo Imaging of Alzheimer Disease β-Amyloid With [11C]SB-13 PET.", American Journal of Geriatric Psychiatry, 2004, 12, p.584-595

[Non-Patent Document 12] Hiroyuki Arai et al., "[11C]-BF-227 AND PET to Visualize Amyloid in Alzheimer's Disease Patients", Alzheimer's & Dementia: The Journal of the Alzheimer's Association, 2006, 2, Sup. 1, S312 [Non-Patent Document 13] Christopher M. Clark et al., "Imaging Amyloid with I123 IMPY SPECT", Alzheimer's & Dementia: The Journal of the Alzheimer's Association, 2006, 2, Sup. 1, S342

[Non-Patent Document 14] D. M. Skovronsky et al., Proc. Natl. Acad. Sci., 2000, 97, 7609

DISCLOSURE OF THE INVENTION

PROBLEMS TO BE SOLVED BY THE INVENTION

As described above, various compounds are disclosed as probes for imaging diagnosis for amyloid, and researched for clinical application.

Experiments in normal mice show that [$^{125}$I]-labeled TZDM, IBOX and m-I-SB are all transferred into brain 2 minutes after administration. However, these compounds are insufficient in clearance from normal tissues, and tend to accumulate gradually in brain as time passes after administration (JP-T-2005-512945; Zhi-Ping Zhuang et al., Nuclear Medicine and Biology, 2001, 28, p.887-894; H. F. Kung et al., J. Am. Chem. Soc., 2001, 123, p.12740-12741). When the clearance from normal tissues is insufficient, a problem arises in that sufficient contrast cannot be obtained at amyloid accumulation sites.

[$^{11}$C]-labeled SB-13 shows a clearance from normal tissues in experiments in rats, however, it cannot be said that the clearance is sufficiently fast (Masahiro Ono et al., Nuclear Medicine and Biology, 2003, 30, p.565-571).

Meanwhile, it is revealed that compounds having an imidazopyridine skeleton such as IMPY have a property of transferring to brain and accumulating at amyloid after administration, and also have an excellent property of rapid clearance from normal tissues unlike the above-described compounds, as a result of experiments using [$^{125}$I] -labeled compounds. However, IMPLY is a compound positive in reverse mutation test. In order to use this compound as a probe for imaging diagnosis, sufficient care must be taken about dosage and administration manner (International Publication No. WO03/106439 pamphlet).

FDDNP is also reported to be positive in reverse mutation test (International Publication No. WO03/106439 pamphlet).

A preferable probe targeting amyloid for imaging diagnosis would be a compound that is excellent in affinity with amyloid and sufficiently rapid in clearance from normal tissues like IMPY but is suppressed in toxicity such as mutagenicity. However, no compound with such properties has been disclosed.

The present invention has been made under such circumstances, and aims at making it possible to detect amyloid *in vivo* or *in vitro* with high sensitivity by providing a compound having affinity with amyloid and suppressed in toxicity such as mutagenicity.

MEANS FOR SOLVING THE PROBLEM

The present inventors have found that a specific novel compound with an imidazopyridine-phenyl skeleton having a carbon atom to which a hydroxyl group is attached has affinity with amyloid and is low in toxicity such as mutagenicity, and amyloid can be detected *in vivo* or *in vitro* with high sensitivity by use of a series of the compound as a probe. Thus, the present invention has been completed.

That is, according to one aspect of the present invention, a reagent for detecting amyloid deposited in a biological tissue is provided, which comprises a compound represented by the following formula (1):

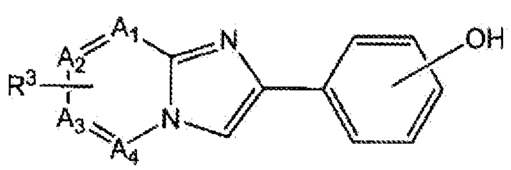

(1)

or a salt thereof.

The biological tissue can be various tissues at which amyloid is known to deposit in amyloidosis.
Typical examples of such biological tissues include brain, heart, lung, pancreas, bone and joint, and as the most typical biological tissue, mention may be made of brain. The typical amyloidosis in case of brain includes Alzheimer's disease and dementia with Lewy bodies.

In the formula (1), $R^3$ is represented by a formula:

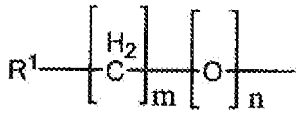

$R^1$ is a radioactive halogen substituent, m is an integer of 0 to 4 and n is 0 or 1. As the radioactive halogen, various nuclides can be used, and preferably a halogen selected from the group consisting of $^{18}F$, $^{75}Br$, $^{76}Br$, $^{123}I$, $^{124}I$, $^{125}I$ and $^{131}I$ can be used, and more preferably a halogen selected from the group consisting of $^{18}F$, $^{123}I$ and $^{125}I$ can be used. Meanwhile, in the formula (1), when n = 0, m = 1 to 4 is preferable, and when n = 1, m = 1 to 4 is preferable.

$A^1$, $A^2$, $A^3$ and $A^4$ independently represent a carbon or nitrogen, and it is necessary that at least one of these represents a carbon. Preferably, 3 or more of $A^1$, $A^2$, $A^3$ and $A^4$ represent carbons, and more preferably, all of them represent carbons. In the formula (1), $R^3$ binds to a carbon represented by $A^1$, $A^2$, $A^3$ or $A^4$. When $A^1$, $A^2$, $A^3$ and $A^4$ respectively represent a carbon which is not bound to $R^3$, a hydrogen atom binding thereto is to be unsubstituted. A hydroxyl group indicated in the formula (1) may bind to any of the carbons constituting the phenyl skeleton thereof, but it is preferable that the hydroxyl group binds to a carbon at 4'-position of the phenyl skeleton. A binding site for $R^3$ may be any of $A^1$, $A^2$, $A^3$ or $A^4$ as long as it is a carbon, but is preferably a carbon represented by $A^3$, that is, a carbon at 6-position.

The compound of the above formula (1) is a nobel compound, and according to another aspect of the present invention, a process for producing a radioactive halogen labeled organic compound is provided, which comprises a step of preparing a reaction solution containing, together with a radioactive halogen ion, a compound represented by the following formula (2):

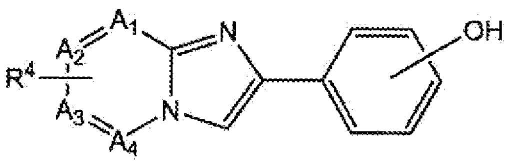

(2)

wherein, $A^1$, $A^2$, $A^3$ and $A^4$ independently represent a carbon or nitrogen, $R^4$ is a group represented by the formula:

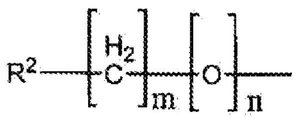

m is an integer of 0 to 4,

n is an integer of 0 or 1, and

when m = n = 0, $R^2$ is a non-radioactive halogen substituent, nitro substituent, trialkylammonium substituent having from 3 to 12 carbon atoms, trialkylstannyl substituent having from 3 to 12 carbon atoms or triphenylstannyl substituent and, when m≠0 and/or n≠0, it is a non-radioactive halogen substituent, methanesulfonyloxy substituent, trifluoromethanesulfonyloxy substituent or aromatic sulfonyloxy substituent,

provided that at least one of $A^1$, $A^2$, $A^3$ and $A^4$ represents a carbon, and $R^4$ binds to a carbon represented by $A^1$, $A^2$, $A^3$ or $A^4$, or a salt thereof,

and a step of giving a reaction condition to the reaction solution to synthesize a compound represented by the following formula (1):

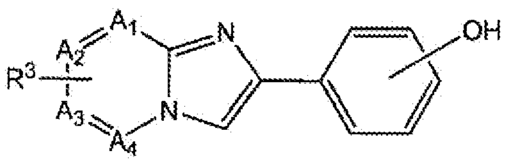

(1)

wherein $A^1$, $A^2$, $A^3$ and $A^4$ are the same as in the formula (2),
$R^3$ is a group represented by the formula:
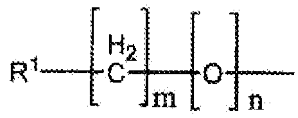

$R^1$ is a halogen substituent,
m and n is the same as in the formula (2),
provided that at least one of $A^1$, $A^2$, $A^3$ and $A^4$ represents a carbon and $R^3$ binds to a carbon represented by $A^1$, $A^2$, $A^3$ or $A^4$, or a salt thereof.

In the formula (2), $A^1$, $A^2$, $A^3$ and $A^4$ independently represent a carbon or nitrogen, but it is necessary that at least one of these represents a carbon. Preferably, 3 or more of $A^1$, $A^2$, $A^3$ and $A^4$ represent carbons, and more preferably, all of them represent carbons. In the formula (2), $R^4$ binds to a carbon represented by $A^1$, $A^2$, $A^3$ or $A^4$. When $A^1$, $A^2$, $A^3$ and $A^4$ respectively represent a carbon which is not bound to $R^4$, a hydrogen atom binding thereto is to be unsubstituted. A hydroxyl group indicated in the formula (2) may bind to any of the carbons constituting the phenyl skeleton thereof, but it is preferable that the hydroxyl group binds to a carbon at 4'-position of the phenyl skeleton. A binding site for $R^4$ is not particularly limited as long as it is a carbon represented by $A^1$, $A^2$, $A^3$ or $A^4$, but is preferably a carbon represented by $A^3$, that is, a carbon at 6-position.

The step of preparing a reaction solution comprising a precursor compound represented above in the formula (2) or a salt thereof and a radioactive halogen ion can be conducted, for example, by dissolving the precursor compound or a salt thereof in an inert organic solvent, and adding thereto a radioactive halogen ion-containing solution which has been obtained with a known method.
As the inert organic solvent, various solvents which do not have reactivity with the precursor compound or a salt thereof and the radioactive halogen ion can be used, for example, when a radioactive halogen ion to be used is a radioactive iodine, methanol can preferably be used, and when a radioactive halogen ion to be used is a radioactive fluorine, acetonitrile can preferably be used.
The reaction condition to be given for the reaction solution in the step off synthesizing a compound represented above in the formula (1) or a salt thereof is not particularly limited as long as it is a condition in which a reaction of substituting $R^2$ of a compound of the formula (2) with the radioactive halogen ion added to the reaction solution is allowed to proceed, and a known reaction condition that fits kinds of the radioactive halogen ion can be used.

In the process for producing the radioactive halogen-labeled organic compound of the present invention, as the radioactive halogen ion, for Example, $^{18}F$, $^{75}Br$, $^{76}Br$, $^{123}I$, $^{124}I$, $^{125}I$ or $^{131}I$ can be used. When a compound of the formula (1) in which the radioactive halogen substituent represented by $R^1$ is $^{123}I$, $^{124}I$, $^{125}I$ or $^{131}I$ is produced, $^{123}I$ ion, $^{124}I$ ion, $^{125}I$ ion or $^{131}I$ ion is respectively used as the radioactive halogen ion. As a compound of the formula (2), it is preferable to use a compound in which $R^2$ is iodine, bromine, trialkylstannyl substituent having from 3 to 12 carbon atoms or triphenylstannyl substituent when m = n = 0, and a compound in which $R^2$ is iodine when m≠O and/or n≠O, and more preferably a compound in which $R^2$ is iodine, trimethylstannyl substituent, tributylstannyl substituent and triphenylstannyl susbtituent particularly when m = n = 0.
When a compound of the formula (1) in which a radioactive halogen substituent represented by $R^1$ is $F^{18}$ is produced, $F^{16}$ ion is used as a radioactive halogen ion, and as a compound of the formula (2), it is preferable to use a compound in which $R^2$ is nitro substituent or trialkylammonium substituent having from 3 to 12 carbon atoms when m = n = 0, and a compound in which is methanesulfonyloxy substituent, trifluoromethanesulfonyloxy substituent or aromatic sulfonyloxy substituent when m≠0 and/or n≠0, and more preferably a compound in which $R^2$ is trifluorimethane sulfonyloxy substituent or toluene sulfonyloxy substituent particularly when m≠0 and/or n≠0. When a compound of the formula (1) in which a

radioactive halogen substituent represented by $R^1$ is $^{75}Br$ or $^{76}Br$ is produced, $^{75}Br$ ion or $^{76}B$ ion is respectively used as the radioactive halogen ion, and as a compound off the formula (2), a compound in which $R^2$ is bromine is preferably used.

Also, according to still another aspect of the present invention, a precursor compound for preparing a radioactive halogen-labeled organic compound is provided, which is represented by the following formula (2):

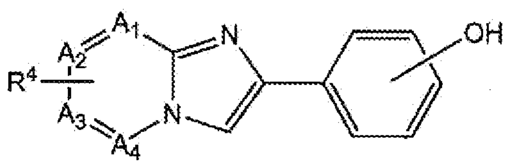

(2)

wherein, $A^1$, $A^2$, $A^3$ and $A^4$ independently represent a carbon or nitrogen,
$R^4$ is a group represented by the formula:

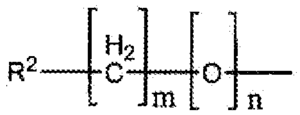

m is an integer of 0 to 4,
n is an integer of 0 or 1,
when m = n = 0, $R^2$ is a non-radioactive halogen substituent, nitro substituent, trialkylammonium substituent having from 3 to 12 carbon, atoms, trialkylstannyl substituent having from 3 to 12 carbon atoms or triphenylstannyl substituent and, when m≠0 and/or n≠0, $R^2$ is a non-radioactive halogen substituent,
methanesulfonyloxy substituent,
trifluoromethanesulfonyloxy substituent or aromatic sulfonyloxy substituent,
provided that at least one of $A^1$, $A^2$, $A^3$ and $A^4$ represents a carbon, and $R^4$ binds to a carbon represented by $A^1$, $A^2$, $A^3$ or $A^4$, or a salt thereof.

As the non-radioactive halogen substituent represented by $R^2$ in the formula (2), a halogen capable of being a target of nucleophilic substitution reactions using a radioactive fluorine or a halogen capable of being a target of isotope exchange reactions with a radioactive iodine can be used, and preferably iodine, bromine or chlorine can be used. As the trialkylstannyl substituent, various substituents can be used, and preferably trimethylstannyl substituent and tributylstannyl substituent can be used. In the formula (2), the preferable $R^2$ is selected from the group consisting of iodine, bromine, trimethylstannyl substituent, tributylstannyl substituent, trifluormethanesulfonyloxy substituent and triphenylstannyl substituent. Meanwhile, in the formula (2), when n = 0, m = 0 to 4 is preferable, and when n = 1, m = 1 to 4 is preferable.

In the formula (2), $A^1$, $A^2$, $A^3$ and $A^4$ independently represent a carbon or nitrogen, but it is necessary that at least one of these represents a carbon. Preferably, or more of $A^1$, $A^2$, $A^3$ and $A^4$ represent carbons, and more preferably, all of them represent carbons. In the formula (2), $R^4$ binds to a carbon represented by $A^1$, $A^2$, $A^3$ or $A^4$. When $A^1$, $A^2$, $A^3$ and $A^4$ respectively represent a carbon which is not bound to $R^4$, a hydrogen atom binding thereto is to be unsubstituted. A hydroxyl group indicated in the formula (2) may bind to any of the carbons constituting the phenyl skeleton thereof, but it is preferable that the hydroxyl group binds to a carbon at 4' -position of the phenyl skeleton. A binding site for $R^4$ may be any of $A^1$, $A^2$, $A^3$ or $A^4$ as long as it is a carbon, but is preferably a carbon represented by $A^3$, that is, a carbon at 6-position.

## EFFECT OF THE INVENTION

In accordance with the present invention, a reagent for detecting amyloid, which has affinity with amyloid and is suppressed in toxicity such as mutagenicity and thus can be used for *in vitro* and *in vivo* detection of amyloid related to a wide range of amyloidosis, can be obtained as well as process for production thereof and a production intermediate thereof.

BEST MODE FOR CARRYING OUT THE INVENTION

(Method for synthesis of a precursor compound for preparing a radioactive halogen-labeled organic compound)

Hereinafter, a method for synthesis of a precursor compound for preparing a radioactive halogen-labeled organic compound according to an aspect of the present invention will be described, taking the case of 6-tributylstannyl-2-(4'-hydroxyphenyl)imidazo[1,2-a]pyridine.

First, 4'-hydroxyacetophenone is allowed to react with cupric bromide to prepare 2-bromo-4'-hydroxyacetophenone (Fig. 1 Step 1). In this instance, the reaction can be conducted in accordance with ordinary methods, for example, the method described in a literature, king, L. Carroll and Ostrum, G. Kenneth, Journal of Organic Chemistry, 1964, 29 (12), p.3459-3461.

Then, 2-brom-4'-hydroxyacetophenone as prepared above is allowed to react with 2-amino-5-bromopyridine to prepare 6-bromo-2-(4'-hydroxy)phenylimidazo[1,2-a]pyridine (Fig. 1, Step 2). This step can be done according to the following procedure.

First, 2-bromo-4'-hydroxyacetophenone and 2-amino-5-bromopyridine are dissolved in an inactive solvent such as acetonitrile, and are allowed to react with each other at a reflux temperature for 2 to 6 hours to produce 6-bromo-2-(4'hydroxyphenyl)imidazo[1,2-a]pyridine hydrobromide salt as white precipitates. The solvent used in this instance may be acetonitrile or another solvent that is usually employed in a similar reaction, for example, methanol and acetone. The reaction temperature may be a temperature allowing reflexing, for example, 90°C when the solvent is acetonitrile. The amount of the solvent to be used may be an amount sufficient to effect the reaction, however, it should be noted that if the solvent is too much, it will become difficult to obtain precipitates of reaction products. For example, when 2-bromo-4'-hydroxyacetophenone in an amount corresponding to 10 mmol is used for the reaction, the amount of a solvent to be used can be about 40 to 50 mL.

Next, the reaction solution is filtered to recover the precipitates. The white precipitates are suspended in a mixed solution of methanol/water (1:1). Then, an aqueous saturated solution of sodium hydrogencarbonate is added thereto in a very excessive amount relative to the precipitates to release 6-bromo-2-(4'-hydroxyphenyl)imidazo[1,2-a] pyridine as precipitates. The newly generated precipitates are filtered to recover 6-brmno-2-(4'-hydroxyphenyl)imidazo [1,2-a]pyridine as the target compound in this step (Fig. 1, Step 2). The amount of the mixed solution of methanol/water is not specifically limited as long was it is 5sufficient to effect the reaction. However, it should be noted that if the amount of the mixed solution is too much, precipitation of products will be hindered. For example, when 2-bromo-4'-hydroxyacetophenone in an amount corresponding to 10 mmol is used, the mixed solution off methanol/water may be used in an amount of about 40 to 100 mL. The amount of sodium hydrogencarbonate is not specifically limited as long as it is very excessive relative to the above-described precipitates as reaction substrates. For example, when the reaction is effected under the above-described conditions, the amount of an aqueous saturated solution of sodium hydrogencarbonate to be added to the reaction solution can be about 25mL.

Then, the 6-bromo-2-(4'-hydroxyphenyl.)imidazo[1,2-a]pyridine prepared above is sufficiently dried, dissolved in dioxane, and after triethylamine is added, bis(tributyltin) and a catalytic amount of tetrakis-triphenylphosphine palladium are added. This reaction mixture is heated at about 90°C and reacted for about 24 hours, and then a solvent is distilled off and a chromatographic purification is performed to obtain 6-tributylstannyl-2-(4'-hydroxyphenyl)imidazo[1,2-a]pyridine as the target compound (Fig. 1, Step 3). The amount of bis(tributyltin) to be used is an amount satisfying a condition where it is excessive relative to the reaction substrate, specifically, it is preferably about 1.5 times in molar ratio relative to the 6-bromo-2-(4'-hydroxyphenyl)imidazo[1,2-a]pyridine as the reaction substrate.

When a compound with a substituent at the 6-position being a trialkylstannyl substituent other than tributylstannyl substituent is obtained, various bis(trialkyltin)s that fit purposes can be used instead of bis(tributyltin) in Step 3. For example, when a compound having a trimethylstannyl substituent as a substituent at the 6-position is synthesized, the same reaction as in the above can be performed in Step 3 using bis(trimethyltin).

In order to obtain a compound with a substituent at the 6-position being a non-radioactive halogen substituent, the compound obtained in Step 2 per se may be used as a compound having bromine as the halogen substituent, and for compounds having fluorine, chlorine and iodine as the halogen substituent at the 6-position, the same reaction as in Step 2 may be performed except using 2-amino-5-fluoropyridine, 2-amino-5-chloropyridine and 2-amino-5-iodopyridine respectively instead of 2-amino-5-bromopyridine in Step 2.

In order to obtain a compound with a substituent at the 6-position being attached thereto via oxygen atom, 2-amino-5-hydroxypyridine instead of 2-amino-5-bromopyridine may be reacted to synthesize 2-(4'-hydroxyphenyl)-6-hydroxyimidazo[1,2-a]pyridine, and a bromide compound having a substituent desired to be introduced may be reacted therewith in the presence of a base. For example, in order to obtain a compound having a 3-fluoropropoxy substituent at the 6-position, 2-(4'-hydroxyphenyl)-6-hydroxyimidazo[1,2-a]pyridine can be reacted with 1-bromo-3-fluoropropane in the presence of potassium carbonate.

Further, in order to obtain a compound with a substituent at the 6-position being attached thereto via an alkyl

chain, the following operations can be performed. For example, for a compound with a substituent at the 6-position being a 3'-bromopropyl group, 2-(4'-hydroxyphenyl)-6-bromoirnidazo[1,2-a]pyridine obtained in Step 2 may be reacted with allyltributyltin, and converted to 6-allyl-2-(4'-hydroxyphenyl)imidazo[1,2-a]pyridine. Then, it is subjected to hydroboronation and oxidation reactions so as to be converted to 2-(4'-hydroxyphenyl)-6-(3'-hydroxypropyl)imidazo[1,2-a]pyridine. Furthermore, bromination of the hydroxyl group by tetrabromomethane may be performed in the presence of triphenylphosphine.

Compounds represented by the above formula (1) in which $A^1$ among $A^1$, $A^2$, $A^3$ and $A^4$ is a nitrogen, and compounds represented by the above formula (2) in which $A^5$ among $A^5$, $A^6$, $A^7$ and $A^8$ is a nitrogen can be produced in accordance with the above method except using 2-amino-5-bromopyrimidine instead of 2-amino-5-bromopyridine in step 2 of Fig. 1.

Compounds represented by the above formula (1) in which $A^3$ and $A^4$ among $A^1$, $A^2$, $A^3$ and $A^4$ are nitrogens, and compounds represented by the above formula (2) in which $A^6$ and $A^8$ among $A^5$, $A^6$ $A^7$ and $A^8$ are nitrogens can be produced in accordance with the above method except using 6-amino-3-bromo-1,2,4-triazine instead of 2-amino-5-bromopyridine in Step 2 of Fig. 1.

(Method for synthesizing a radioactive halogen-labeled organic compound)

Hereinafter, a method for production of a radioactive halogen-labeled organic compound according to another aspect of the present invention will be described by taking the case of 2-(4'-hydroxyphenyl)-6-[$^{123}$I]iodoimidazo[1,2-a]pyridine.

For the production of 2-(4'-hydroxyphenyl)-6- [$^{123}$I]iodoimidazo[1,2-]pyridine, a[$^{123}$I]sodium iodide solution as a radioactive halogen ion to be served for labeling is first obtained. A [$^{123}$I]radioactive iodine can be obtained by, for example, a known method in which a xenon gas is used as a target and exposed to proton bombardment. This [$^{123}$I] radioactive iodine is made into [$^{123}$I]sodium iodide solution by using known methods, and used for the labeling.

Then, the labeling precursor 6-tributylstannyl-2- (4'-hydroxyphenyl)imidazo[1,2-a]pyridine is dissolved in an inert organic solvent, and the [$^{123}$I]sodium iodine solution, an acid and an oxidizing agent are added thereto and allowed to react to obtain 2-(4'-hydroxyphenyl)-6-[$^{123}$I]iodoimidazo[1,2-a]pyridine as a target compound. As the inert organic solvent dissolving the precursor compound, various solvents having no reactivity with the labeling precursor and the [$^{123}$I]sodium iodide can be used, and preferably methanol can be used.

As the acid, may be used various ones, and preferably hydrochloric acid.

The oxidizing agent is not particularly limited as long as it can effect the oxidation of iodine in the reaction solution, and is preferably hydrogen peroxide or peracetic acid. The amount of the oxidizing agent to be added may be an amount sufficient to oxidize iodine in the reaction solution.

A compound labeled with a radioactive halogen other than iodine can be synthesized by labeling a labeling precursor that fits a purpose of synthesis with a radioactive halogen that fits the purpose. For example, in order to synthesize 6-[$^{18}$F]fluoropropoxy-2-(4'-hydroxyphenyl)imidazo[1,2-a]pyridine, the labeling precursor 2-(4'-hydroxyphenyl)-6-(3'-paratoluenesulfonyloxypropoxy)imidazo[1,2-a]pyridine can be reacted with [$^{18}$F]fluoride ion in the pretence of a phase transfer catalyst and potassium carbonate.

(Methods for preparing and using a detection reagent in accordance with the present invention)

Amyloid has many different structures depending upon kinds of precursor protein, but they are common in a point of having β-sheet structure. It has been knows that many staining reagents for amyloid such as Thioflavin T and Congo-red target the β-sheet structure, and have the staining ability equally to different kinds of amyloid.

The compound of the formula (1) according to the present invention has affinity with amyloid originated from amyloid β-protein (hereinafter referred to as Aβ) as a precursor compound, and also has an activity of inhibiting the binding to amyloid of Thioflavin T which is known to bind to a wide range of amyloid.

Therefore, the compound of the formula (1) according to the present invention is considered to have affinity with β-sheet structure of amyloid protein like Thioflavin T. This suggests that the compound of the formula (1) according to the present invention has the same affinity with various amyloid.

That is, the reagent for detecting amyloid according to the present invention can be used for diagnosing the systemic and localized amyloidosis similarly to Thioflavin T and Congo-red. The systemic amyloidosis includes immunoglobulin amyloidosis, reactive AA amyloidosis, familial amyloidosis, dialysis amyloidosis and senile amyloidosis. The localized amyloidosis includes brain amyloidosis, endocrine amyloidosis, cutaneous amyloidosis and localized nodular amyloidosis.

The reagent for detecting amyloid according to the present invention can be not only used as a reagent used *in vitro* for biopsy, but also used as a reagent used *in vivo* as a radioactive diagnostic agent.

The reagent for detecting amyloid according to the present invention can be prepared as a solution which

comprises the radioactive halogen-labeled compound of the above formula (1) blended in water, a physiological saline solution or a Ringer's solution optionally adjusted to an appropriate pH, like other commonly-known radioactive diagnostic agents. In this instance, concentration of the present compound should be adjusted to not more than the concentration at which stability of the present compound is ensured. Dosage of the present compound is not specifically limited as long as it is sufficient to obtain an image of distribution of an administered agent. For example, in case of iodine-123-labeled compounds and fluorine-18-labeled compounds, about 50 to 600 MBq per adult body of 60 kg weight can be administered intravenously or locally. Distribution of administered agents can be image by known methods. For example, iodine-123-labeled compounds can be imaged by a SPECT apparatus while fluorine-18-labeled compounds can be imaged by a PET apparatus.

By administering the reagent for detecting amyloid according to the present invention to a living body, amyloid which is deposited in biological tissues such as brain, heart, lung, digestive tract, blood vessel, liver, pancreas, kidney, joints and bones can be imaged, and it is useful for imaging amyloid deposition in biological tissues difficult in biopsy collection, for example, brain, heart, lung, pancreas, bone and joint.

## EXAMPLE

Hereinafter, the present invention is described below in more detail by way of Examples, Comparative Examples and Reference Examples. However, these Examples never limit the scope of the present invention.

In the following Examples, the names of the individual compounds used in the experiment are defined as shown in Table 1.

Table 1: Names of compounds used for evaluation in Examples

| Compound name | Common name |
|---|---|
| Compound 1 | 6-bromo-2-'4'hydroxyphenyl)imidazo[1,2-a]pyridine |
| Compound 2 | 2-(4'-hydroxypheuyl)-6-iodoimidazo[1,2- a]pyridine |
| Compound 3 | 6-(3'-fluoropropoxy)-2-(4'-hydroxyphenyl)imidazo[1,2-a]pyridine |
| Compound 4 | 2-(4'-hydroxyphenyl)-6-iodoimidazo[1,2- a]pyrimidine |
| Compound 5 | [$^{125}$I]-2-(4'-hydroxyphenyl)-6-iodoimdazo[1,2-a]pyridine |
| Compound 6 | [$^{123}$I]-2-(4'-hydroxyphenyl)-6-iodoimdazo[1,2-a]pyridine |
| Compound 7 | 6-bromo'-2'-(4'-hydroxyphenyl)imidazo[1,2-a]pyrazine |
| Compound 8 | 2-(4'-hydroxyphenyl)-6-iodoimidazo[1,2-a]pyrazine |
| Compound 9 | [$^{123}$I]-2-(4'-hydroxyphenyl)-6-iodoimidazo[1,2-a]pyrimidine |
| Compound 10 | [$^{123}$I]-2-(4'-hydroxyphenyl)-6-iodoimidazo[1,2-a]pyrazine |
| Compound 11 | [$^{123}$I]-2-(4'-hydroxyphenyl)-8-iodoimidazo[1,2-a]pyridine |

(Example I-1) Synthesis of 6-tributylstannyl-2-(4'-hydroxyphenyl)imidazo[1,2-a]pyridine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elation solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 1, step 1).

2.15 g (corresponding to 10.0 mmol) of 2-bromo-4'-hydroxyacetophenone and 1.74 g (corresponding to 10.0 mmol) of 2-amino-5-bromopyridine were dissolved in 50 mL of acetonitrile. The resulting solution was was refluxed in an oil bath at 105°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture

was sonicated for 5 minutes using an ultrasonic washing machine. Precipitates were filtered and recovered from the resulting mixture, sufficiently washed with water, and dried under reduced pressure, to obtain 2.41 g (corresponding to 8.32 mmol) of 6-bromo-2-(4'-hydroxyphenyl)imidazo[1,2-a]pyridine (Fig. 1, Step 2).

138 mg (corresponding to 0.476 mmol) of 6-bromo-2-(4'-hydroxyphenyl)imidazo[1,2-a]pyridine was dissolved in 20 mL of dioxane, and 2 mL of triethylamine was added thereto. Then, 360 μL (corresponding to 0.713 mmol) of bis (tributyltin) and 20 mg (at a catalytic amount) of tetrakis-triphenylphosphine were added. After the reaction mixture was stirred at 90°C for 22 hours, the solvent was distilled off under reduced pressure. The residue was purified by preparative TLC (elution solvent: hexane/ethyl acetate = 1/4). Further, the resulting crude product was purified by recycle preparative HPLC (HPLC apparatus: LC-908 (under trade name; manufactured by Nippon Bunseki Kogyo); column: two of JAIGEL 2H (under trade name; manufactured by Nippon Bunseki Kogyo) connected together; mobile phase: chloroform), to obtain 47 mg (corresponding to 94.9 μmol) of 6-tributylstannyl-2-(4'-hydroxyphenyl)imidazo[1,2-a]pyridine (Fig. 1, Step 3).

The NMR measurement results of the resulting 6-tributylstannyl-2-(4'-hydroxyphenyl)imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 8.01-7.94 (m, 1H), 7.71-7.67 (m, 2H), 7.70-7.67 (m, 1H), 7.64-7.60 (m, 1H), 7.20-7.11 (m, 1H), 6.89-6.85 (m, 2H), 1.62-1.46 (m, 6H), 1.34 (sext, J = 7.3 Hz, 6H), 1.18-1.03 (m, 6H), 0.90 (t, J = 7.3 Hz, 9H).

$^{13}$C-NMR (solvent: chloroform-dl, resonance frequency: 125 NHz): δ 157.85, 145.11, 144.72, 131.90, 129.93, 127.62, 124.02, 122.59, 116.14, 116.09, 106.19, 28.96, 27.27, 13.62, 9.81.

(Example I-2) Synthesis of [$^{125}$I]-2-(4'-hydroxyphenyl)-6-iodoimidazo[1,2-a]pyridine

To 53 μL of a solution of 6-tributylstannyl-2-(4'-hydroxyphenyl)imidazo[1,2-a]pyridine in methanol (concentration: 1 mg/mL), 75 μl of 1 mol/L hydrochloric acid, [$^{125}$I] sodium iodide of 136 MBq (40 μL in volume) and 10 μL of 10% (w/v) hydrogen peroxide were added. After the mixed solution was left to stand at 50°C for 10 minutes, the solution was subjected to HPLC under the following conditions, to obtain [$^{125}$I]-2-(4'-hydroxyphenyl)-6-iodoimidazo[1,2-a]pyridine fraction.

HPLC conditions:

Column: Phenomenex Luna C18 (trade name; manufactured by Phenomenex Co.; size: 4.6 x 150 mm)
Mobile phase: 0.1 % trifluoroacetic acid in water/0.1 % trifluoroacetic acid in acetonitrile = 80/20 to 0/100 (17 minutes, linear gradient)
Flow rate: 1.0 mL/min.
Detector: Ultraviolet visible absorptiometer (Detection wavelength: 282 nm) and radioactivity counter (manufactured by raytest: type STEFFI)

10 ml of water was added to the fraction. The resulting solution was passed through a reversed phase column (trade name: Sep-Pak (registered trademark, Waters Investments Limited) Light C18 Cartridges manufactured by Waters: the packed amount of the agent: 130 mg) so that the column adsorbs and collects [$^{125}$I]-2-(4'-hydroxyphenyl)-6-iodoimidazo[1,2-a]pyridine. The column was rinsed with 1 mL of water, and then 1 mL of ethanol was passed therethrough to elute [$^{125}$I]-2-(4'-hydroxyphenyl)-6-iodoinidazo[1,2-a]pyridine. The amount of radioactivity of the obtained compound was 37.5 MBq immediately after the synthesis. Further, the TALC analysis was conducted under the following conditions, and as a result, the radiochemical purity of the compound was 96.5%.

TLC analysis conditions:

TLC plate: RP-18F254 (trade name; manufactured by Merck & Co., Inc.)
Mobile phase: Methanol/water = 20/1
Detector: Bio-imaging Analyzer, BAS-2500 (type: BAS-2500 manufactured by FUJIFILM Corporation)

(Example I-3) Synthesis of [$^{123}$I]-2-(4'-hydroxyphenyl)-6-iodoimidazo[1,2-a]pyridine

To 70 μL of a solution of 6-tributylstannyl-2-(4'-hydroxyphenyl)imidazo[1,2-a]pyridine in methanol (concentration: 1 mg/mL), 75-100 μL of 1 mol/L hydrochloric acid, [$^{123}$I]sodium iodide of 236-454 MBq (15-120 μL in volume) and 7.5-10 μL of 1 mmol/L sodium iodide solution and 10-15 μL of 10% (w/v)hydrogen peroxide were added. After the mixed solution was heated at 50°C for 10 minutes, the solution was subjected to HPLC under the same conditions as in Example I-2, to obtain [$^{123}$I]-2-(4'-hydroxyphenyl)-6-iodoimidazo[1,2-a]pyridine as a fraction.

10 ml of water was added to the fraction. The resulting solution was passed through a reversed phase column

(trade name: Sep-Pak (registered trademark, caters Investments Limited) Light C18 Cartridge manufactured by Waters: the packed amount of the packing agent: 130 mg) so that the column adsorbs and collects [$^{123}$I]-2-(4'-hydroxyphenyl)-6-iodoimidazo[1,2-a]pyridine. The column was rinsed with 1 m of water, and then 1 mL of diethyl ether was passed therethrough, to elute [$^{123}$I]-2-(4'-hydroxyphenyl)-6-iodoimidazo[1,2-a]pyridine. The amount of radioactivity of the obtained compound was 21-180 MBq immediately after the synthesis. Further, the TLC analysis was conducted under the same conditions as in Example I-2, and as a result, the radiochemical purity of the compound was 99.5%.

(ExampleI-4) Synthesis of 6-bromo-2-(4'-hydroxyphenyl)imidazo[1,2-a]pyridine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addition off active charcoal. Then, the resulting solution was filtered and concentrated. The resulting crude product was purified by flash silica gel column chromatography (elution solvent: chloroform/methanol = 20/1), and recrystallized from ethyl acetate/petroleum ether to obtain 7.25 g (corresponding to 33.7 mmol) of 2-bromo-4'-hydroxyacetophenone (Fig. 2, Step 1).

2.15 g (corresponding to 10.0 mmol) of 2-bromo-4'-hydroxyacetophenone and 1.74 g (corresponding to 10.0 mmol) of 2-amino-5-bromopyridine were dissolved in 50 mL of The resulting solution was refluxed in an oil bath at 105°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 20 mL of water and 20 mL of methanol. Then, about 25 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 2.41 g (corresponding to 8.32 mmol) of 6-bromo-2-(4'-hydroxyphenyl)imidazo[1,2-a]pyridine (Fig. 2, Step 2).

The NMR measurement results of the resulting 6-bromo-2-(4'-hydroxyphenyl)imidazo[1,2-a]pyridine (internal standard: dimethylsulfoxide) are shown below.

NMR apparatus employed: JNM-ECP-500 (manufactured by Japan Electron Optics Laboratory Co., Ltd. (JEOL)) $^{1}$H-NMR (solvent: dimethylsulfoxide-d6, resonance frequency: 500 MHz): δ 9.54 (br. s, 1H), 8.83-8.81 (m, 1H), 8.17 (s, 1H), 7.79-7.74 (m, 2H), 7.51 (d, J = 9.6 Hz, 1H), 7.30 (dd, J = 9.6, 1.8 Hz, 1H), 6.86-6.81 (m, 2H).

$^{13}$C-NMR (solvent: dimethylsulfoxide-d6, resonance frequency: 125 MHz): δ 158.15, 146.40, 143.79, 127.82, 127.67, 127.14, 125.01, 117.87, 116.15, 103.60, 106.05.

(ExampleI-5) Synthesis of 2-(4'-hydroxyphenyl)-6-iodoimidazo[1,2-a]pyridine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol - 20/1), and recrystallized from ethyl acetate/petroleum ether, to obtain 7.25 g (corresponding to 33.7 mmol) of 2-bromo-4'-hydroxyacetophenone (Fig. 3, Step 1).

441 mg (corresponding to 2.0 mmol) of 2-bromo-4'-hydroxyacetophenone and 449 mg (corresponding to 2.0 mmol) of 2-amino-5-iodopyridine were dissolved in 15 mL of acetonitrile. The resulting solution was refluxed in an oil bath at 110°C for 5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 526 mg (corresponding to 1.56 mmol) of 2-(4'-hydroxyphenyl)-6-iodoimidazo[1,2-a]pyridine (Fig. 3, Step 2).

The NMR measurement results of the resulting 2-(4'-hydroxyphenyl)-6-iodoimidazo[1,2-a]pyridine (internal standard: dimethylsulfoxide) are shown below.

NMR apparatus employed: JNM-ECP-500 (manufactured by Japan Electron Optics Laboratory Co., Ltd. (JEOL)) $^{1}$H-NMR (solvent: dimethylsulfoxide-d6, resonance frequency: 500 MHz): δ 8.86-8.84 (m, 1H), 8.14 (s, 1H),

7.78-7.74 (m, 2H), 7.40-7.35 (m, 2H), 6.86-6.82 (m, 2H).

$^{13}$C-NMR (solvent: dimethylsulfoxide-d6, resonance frequency: 125 MHz): δ 158.08, 145.87, 143.87, 132.48, 131.72, 127.67, 124.99, 113.14, 116.14, 108.02, 75.85.

(ExampleI-6) Synthesis of 2-(4'-hydroxyphenyl)-6-iodoimidazo[1,2-a]pyrimidine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 4, Step 1).

646 mg (corresponding to 3.0 mmol) of 2-bromo-4'-hydroxyacetophenone and 668 mg (corresponding to 3.0 mmol) of 2-amino-5-iodopyrimidine were dissolved in 20 mL of acetonitrile. The resulting solution was refluxed in an oil bath at 110°C for 8 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 15 mL of a saturated sodium hydrogencarbonate solution was added thereto, and the mixture was sonicated for 3 minutes using an ultrasonic washing machine. Precipitates were filtered and recovered from the resulting mixture, sufficiently washed with water, and dried under reduced pressure, to obtain 737 mg (corresponding to 2.19 mmol) of 2-(4'-hydroxyphenyl)-6-iodoimidazo[1,2-a]pyrimidine (Fig. 4, Step 2).

The NMR measurement results of the resulting 2-(4'-hydroxyphanyl)-6-iodoimidazo[1,2-a]pyrimidine (internal standard: dimethylformamide) are shown below.

NMR apparatus employed: JNM-ECP-500 (manufactured by Japan Electron Optics Laboratory Co., Ltd. (JEOL))
$^{1}$H-NMR (solvent: dimethylformamide-d7, of resonance frequency: 500 MHz): δ 9.80 (br. s, 1H), 9.35 (d, J = 2.3 Hz, 1H), 8.60 (d, J = 2.3 Hz, 1H), 8.23 (s, 1H), 7.94-7.90 (m, 2H), 6.98-6.94 (m, 2H).

$^{13}$C-NMR (solvent: dimethylformamide-d7, resonance frequency: 125 MHz): δ 153.87, 154.00, 147.18, 146.77, 139.07, 127.68, 124.50, 115.85, 106.10, 73.46.

(Example I-7) Synthesis of 6-(3'-fluoropropoxy)-2-(4'-hydroxyphenyl)imidazo[1,2-a]pyridine

31.11 g (corresponding to 178.88 mmol) of 2-bromo-3-hydroxypyridine was dissolved in 95.8 mL of dimethylsulfoxide, and 89.9 mL (corresponding to 89.9 mmol) of 1 mol/L sodium methoxide-methanol solution was added thereto. Then, the reaction solution was heated to 90°C to distill off methanol. After the reaction solution was cooled down to 5°C or lower, 29.2 g (corresponding to 205.62 mmol) of methyl iodide was added, and then stirred at room temperature for 17 hours. After the completion of the reaction, the reaction solution was poured into ice water and extracted twice with chloroform. The combined chloroform layer was washed with 1 mol/L sodium hydroxide solution, washed twice with a saturated sodium chloride solution, and dried over anhydrous sodium sulfate. After the solvent was distilled off under reduced pressure, 20.74 g (corresponding to 110.31 mmol) of 2-bromo-3-methoxypyridine was obtained (Fig. 5, Step 1).

83 mL of conc. sulfuric acid was cooled down to -5°C, and 83 mL of 90 % nitric acid was carefully added thereto. Subsequently, 20.69 g (corresponding to 110.04 mmol) of 2-bromo-3-methoxypyridine was carefully added thereto. After the reaction mixture was stirred in an ice bath for 5 minutes, the mixture was stirred at room temperature for 10 minutes, and then was heated to 55°C and further stirred for an hour. After the reaction solution was cooled to room temperature, the reaction solution was poured little by little into crushed ice to generate precipitates. The precipitates were filtered and washed with water, and then dried over phosphorous pentoxide under reduced pressure, to obtain 17.41 g (corresponding to 74.71 mmol) of 2-bromo-3-methoxy-6-nitropyridine (Fig. 5, Step 2).

17.36 g (corresponding to 74.50 mmol) of 2-bromo-3-methoxy-6--nitropyridine was dissolved in 520 mL of ethanol, and 11.63 g (50% wet) of 10 % palladium-carbon was added thereto under argon stream. To the mixture, 88.4 mL of hydrazine monohydrate was added dropwise. After the reaction mixture was refluxed for 45 minutes, the reaction solution was cooled down to room temperature. Then, after palladium-carbon was filtered off, the residue was washed with ethanol, and the washing were combined with the filtrate. The combined solution was concentrated under reduced pressure. Then, 402 mL of water and 38 mL of conc. aqueous ammonia were added to the concentrate, and the resulting mixture was extracted eight times with chloroform. The combined chloroform layer was dried over anhydrous sodium sulfate and concentrated under reduced pressure. The resulting crude product was distilled under reduced pressure to obtain 8.14 g (corresponding to 65.57 mmol) of 2-amino-5-methoxypyridine (Fig. 5, Step 3).

13.50 g (corresponding to 59.66 mmol) of 4'-benzoyloxyacetophenone was dissolved in 1100 ml of methanol, and 34.52 g (corresponding to 71.59 mmol) of tetra-n-butylammonium tribromide was added thereto. The mixture was stirred oversight at room temperature, and was distilled off under reduced pressure to remove the solvent. The residue was dissolved in ethyl acetate and washed twice with water and then washed with an aqueous saturated sodium chloride solution. After the ethyl acetate layer was dried over anhydrous sodium sulfate and concentrated under reduced pressure, the resulting crude product was purified by silica gel column chromatography (elution solvent: hexane/methylene chloride = 1/1) , to obtain 13.38 g (corresponding to 43.84 mmol) of 4'-benzoyloxy-2-bromoacetophenone (Fig. 5, Step 4).

13.33 g (corresponding to 43.68 mmol) of 4'-benzoyloxy-2-bromoacetophenone and 5.67 g (corresponding to 45.67 mmol) of 2-amino-5-methoxypyridine were dissolved in 481 mL of ethanol. The resulting solution was refluxed for 2 hours. After the reaction solution was cooled, 6.64 g (corresponding to 79.09 mmol) of sodium hydrogencarbonate was added thereto. The resulting reaction mixture was further refluxed for 4 hours. After the completion of the reaction, the solvent was concentrated under reduced pressure. The resulting residue was dissolved in chloroform and then washed with water. The chloroform layer was dried over anhydrous sodium sulfate, the solvent was distilled off. The resulting crude product was purified by silica gel column chromatography (elution solvent: chloroform/ethyl acetate = 20/1), to obtain 10.20 g (corresponding to 30.87 mmol ) of 2-(4'-benzoyloxyphenyl)-6-methoxyimidazo[1,2-a]pyridine (Fig. 5, Step 5).

4.90 g (corresponding to 14.83 mmol) of 2-(4'-benzoyloxyphenyl)-6-methoxyimidazo[1,2-a]pyridine that was sufficiently dried to remove moisture was dissolved in 245 mL of chloroform and cooled down to -15°C. To this solution, a solution of 12.62 mL (corresponding to 133.43 mmol) of boron tribromide in 134 mL of dichloromethane was added dropwise. After the temperature of the resulting solution was raised to room temperature, the solution was stirred for 17 hours. After the completion of the reaction, the reaction solution was cooled with ice and supplemented with 668 mL of methanol, and further stirred at room temperature for 3 hours. The reaction mixture was then concentrated under reduced pressure. The resulting crude product was supplemented with 290 mL of chloroform and 29 mL of methanol to obtain slurry, and then precipitates were filtered and recovered. The precipitates recovered were washed with chloroform and then dried under reduced pressure, to obtain 3.00 g (corresponding to 13.28 mmol) of 2-(4'-bydroxyphenyl)-6-hydroxyimidazo[1,2-a]pyridine (Fig. 5, Step 6).

560 mg (corresponding to 2.48 mmol) of 2-(4'-hydroxyphenyl)-6-hydroxyimidazo[1,2-'a]pyridine was dissolved in 21 mL of dimethylformamide, and 1.37 g (corresponding to 9.90 mmol) of potassium carbonate and 349 mg (corresponding to 2.48 mmol) of 1-bromo-3-fluoropropane were added thereto. The solution was stirred at room temperature for 24 hours. The reaction solution was concentrated under reduced pressure, and then supplemented with 10 mL of chloroform and 10 mL of methanol to obtain slurry. The slurry was filtered and filtrate was concentrated. The resulting crude product was purified by silica gel column chromatography (elution solvent: chloroform/methanol = 20/1), to obtain 151 mg (corresponding to 0.527 μmol) of 6-(3'-fluoropropoxy)-2-, (4'-hydroxyphenyl)imidazo[1,2-a]pyridine (Fig. 5, Step 7).

The NMR measurement results of the resulting 6-(3'-fluoropropoxy)-2-(4'-hydroxyphenyl)imidazo[1,2-a]pyridine (internal standard: dimethylsulfoxide) are shown below.

NMR apparatus employed: JNM-GSX-270 (manufactured by Japan Electron Optics Laboratory Co., Ltd. (JEOL))

$^1$H-NMR (solvent: dimethylsulfoxide-d6; resonance frequency: 270 MHz): δ 9.52 (s, 1H), 8.22(d, J = 2.2 Hz, 1H), 8.08 (s, 1H), 7.75-7.65 (m, 2H), 7.44 (d, J = 9.6 Hz, 1H), 6.99 (dd, J = 9.6, 2.2 Hz, 1H), 6.85-6.75 (m, 2H), 4.62 (dt, $^2J_{HE}$ = 47.0 Hz, J = 6.0 Hz, 2H), 4.05 (t, J = 6.0 Hz, 2H), 2.13 (dquint, $^3J_{HE}$ = 25.9 Hz, J = 6.0 Hz, 2H).

(ExampleI-8) Synthesis of 6--bromo-2-(4'-hydroxyphenyl)imidazo[1,2-a]pyrimidine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction mixture cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 6, Step 1).

748 mg (corresponding to 3.5 mmol) of 2-bromo-4'-hydroxyacetophenone and 605 mg (corresponding to 3.5 mmol) of 2-amino-5-bromopyrimidine were dissolved in 30 mL of acetonitrile. The resulting solution was refluxed in an oil bath at 110°C for 5 hours. After the completion of the reaction, the reaction solution was cooled down to room temperature, and precipitates were filtered and recovered. The precipitates were washed with any dried under reduced pressure. The resulting crude crystals were suspended in a mixed solution of 10 mL of water and 15 mL of methanol. Then, about 10 mL of a saturated sodium hydrogencarbonate solution was added thereto, and the mixture was sonicated

for 5 minutes using an ultrasonic washing machine. Precipitates were filtered and recovered from the resulting mixture, sufficiently washed with water, and dried under reduced pressure. The resulting crude product was recrystallized from N,N-dimethylformamide, to obtain 289 mg (corresponding to 0.997 mmol) of 6-bromo-2-(4'-hydroxyphenyl)imidazo[1,2-] pyrimidine (Fig. 6, Step 2).

The NMR measurement results of the resulting 6-bromo-2-(4'-hydroxyphenyl)imidazo[1,2-a]pyrimidine (internal standard: dimethylsulfoxide) are shown below.

NMR apparatus employed: JNM-ECP-500 (manufactured by Japan Electron Optics Laboratory Co., Ltd. (JEOL)) $^1$H-NMR (solvent: dimethylsulfoxide-d6, resonance frequency: 500 MHz): δ 9.56 (br. s, 1H), 9.21 (d, J = 2.5 Hz, 1H), 8.46 (d, J = 2.5 Hz, 1H), 8.09 (s, 1H), 7.79-7.75 (m, 2H), 6.83-6.79 (m, 2H).

$^{13}$C-NMR (solvent: dimethylsulfoxide-d6, resonance frequency: 125 MHz): δ 158.63, 149.99, 147.68, 146.88, 134.78, 127.93, 124.52, 116.23 106.83, 103.94.

(ExampleI-9) Synthesis of 6-fluoro-2-(4'-hydroxyphenyl)imidazo[1,2-a]pyridine

70 mL of ethyl acetate was added to 40.0 g (corresponding to 179 mmol) of cupric bromide to obtain a suspension, to which a solution of 11.6 g (corresponding to 85.3 mmol) of 4'-hydroxyacetophenone in a mixed solution of 70 mL of ethyl acetate and 70 mL of chloroform was added. Then, the resulting mixture was refluxed. After 5.5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/ petroleum ether, to obtain 10.2 g (corresponding to 47.3 mmol) of 2-bromo-4'-hydroxyacetophenone (Fig. 7, Step 1).

439 mg (corresponding to 2.0 mmol) of 2-bromo-4'-hydroxyacetophenone and 224 mg (corresponding to 2.0 mmol) of 2-amino-5-fluoropyridine were dissolved in 20 mL of acetonitrile. The resulting solution was refluxed in an oil bath at 110°C for 5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 8 mL of water and 8 mL of methanol. Then, about 10 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 302 mg (corresponding to 1.32 mmol) of 6-fluoro-2-(4'-hydroxyphenyl)imidazo[1,2-a]pyridine (Fig. 7, Step 2).

The NMR measurement results of the resulting 6-fluoro-2-(4'-hydroxyphenyl)imidazo[1,2-a]pyridine (internal standard: dimethylsulfoxide) are shown below.

NMR apparatus employed JNM-ECP-500 (manufactured by Japan Electron Optics Laboratory Co., Ltd. (JEOL)) $^1$H-NMR (solvent: dimethylsulfoxide-d6, resonance frequency: 500 MHz): δ 9.45 (br. s, 1H), 8.65 (ddd, $^3J_{HF}$ = 4.6 Hz, J = 2.5, 0.7 Hz, 1H), 8.16-8.15 (m, 1H), 7.75-7.69 (m, 2H), 7.56-7.51 (m, 1H), 7.23 (ddd, $^3J_{HF}$ = 8.4 Hz, J = 9.9, 2.5 Hz, 1H), 6.82-6.76 (m, 2H).

$^{13}$C-NMR (solvent: dimethylsulfoxide-d6, resonance frequency: 125 MHz): δ 157.82, 152.81 (d, $^1J_{CF}$ = 232.3 Hz), 146.58, 142.92, 127.35, 124.99, 117,19 (d, $^3J_{CF}$ = 9.6 Hz), 116.40 (d, $^2J_{CF}$ = 25.9 Hz), 115.89, 113.66 (d, $^2J_{CF}$ = 41.8 Hz), 109.48.

$^{19}$F-NMR (solvent: dimethylsulfoxide-d6, resonance frequency: 470 MHz): δ 141.93 (br. s).

(ExaropleI-10) Synthesis of 2-(4'-hydroxyphenyl)-6-nitroimidazo[1,2-a]pyridine

70 mL of ethyl acetate was added to 40.0 g (corresponding to 179 mmol) of cupric bromide to obtain a suspension, to which a solution of 11.6 g (corresponding to 85.3 mmol) of 4'-hydroxyacetophenone in a mixed solution of 70 mL of ethyl acetate and 70 mL of chloroform was added. Then, the resulting mixture was refluxed. After 5.5 hours, the reaction mixture was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/ petroleum ether, to obtain 10.2 g (corresponding to 47.3 mmol) of 2-bromo-4'-hydroxyacetophenone (Fig. 8, Step 1).

432 mg (corresponding to 2.0 mmol) of 2-bromo-4'-hydroxyacetophenone and 279 mg (corresponding to 2.0 mmol) of 2-amino-5-nitropyridine were dissolved in 20 mL of acetonitrile. The resulting solution was refluxed in an oil bath at 110°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 8 mL of water and 8 mL of methanol. Then, about 8 mL of a saturated sodium hydrogencarbonate solution was added thereto, and the mixture was sonicated for 3

minutes using an ultrasonic washing machine. Precipitates were filtered and recovered from the resulting mixture, sufficiently washed with water, and dried under reduced pressure, to obtain 148 mg (corresponding to 0.580 mmol) of 2-(4'-hydroxyphenyl)-6-nitroimidazo[1,2-a]pyridine (Fig. 8, Step 2).

The NMR measurement results of the resulting 2-(4'-hydroxyphenyl)-6-nitroimidazo[1,2-a]pyridine (internal standard: dimethylsulfoxide) are shown below.

NMR apparatus employed: JNM-ECP-500 (manufactured by Japan Electron Optics Laboratory Co., Ltd. (JEOL))
$^{1}$H-NMR (solvent: dimethylsulfoxide-d6, resonance frequency: 500 MHz): δ 9.74-9.72 (m, 1H), 9.59 (br. s, 1H), 8.39 (s, 1H), 7.87 (dd, J = 9.9, 2.3 He, 1H), 7,79-7.74 (m, 2H), 7.65-7.61 (m, 1H), 6.84-6.80 (m, 2H).

$^{13}$C-NMR (solvent: dimethylsulfoxide-d6, resonance frequency: 125 MHz) : δ 158.47, 148.51, 145.25, 136.63, 127.93, 127.81, 124.06, 118.92, 116.09, 115.92, 110.37.

(Example II-1) Synthesis of 6-tributylstannyl-2-(4'-hydroxyphenyl)imidazo[1,2-a]pyrimidine

50 mL of ethyl acetate was added to 28.17 g (corresponding to 126 mmol) of cupric bromide to obtain a suspension, to which a solution of 8.18 g (corresponding to 60.0 mmol of 4'-hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum, ether, to obtain 7.25 g (corresponding to 33.7 mmol) of 2-bromo-4'-hydroxyacetophenone (Fig. 10, Step 1).

748 mg (corresponding to 3.48 mmol) of 2-bromo-4'-hydroxyacetophenone and 605 mg (corresponding to 3.48 mmol) of 5-bromo-2-aminopyrimidine were dissolved in 30 mL of acetonitrile. The resulting solution was refluxed in an oil bath at 110°C for 5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 15 mL of methanol. Then, about 10 mL of a saturated sodium hydrogencarbonate solution was added, thereto, and the mixture was sonicated for 5 minutes using an ultrasonic washing machine. Precipitates were filtered, and recovered from the resulting mixture, sufficiently washed with water, and dried under reduced pressure. The obtained solid was recrystallized from DMF, to obtain 289 mg (corresponding to 1.00 mmol) of 6-bromo-2-(4'-hydroxyphenyl)imidazo[1,2-a]pyrimidine (Fig. 10, Step 2).

75.4 mg (corresponding to 0.260 mmol) of 6-bromo-2-(4'-hydroxyphenyl)imidazo[1,2-a]pyrimidine was dissolved in 10.0 mL of dioxane, and 2.0 mL of triethylamine was added thereto. Then, 0.20 mL (corresponding to 0.39 mmol) of bis(trioutyltin) and 20.1 mg (at a catalytic amount) of tetrakis-triphenylphosphine palladium were added thereto. After the reaction mixture was stirred at 90°C for 10 hours, the solvent was distilled off under reduced pressure. The residue was purified by flash silica gel column chromatography (elution solvent: hexane/ethyl acetate = 1/1), to obtain 24.0 mg (corresponding to 0.048 mmol) of 6-tributylstannyl-2-(4'-hydroxyphenyl)imidazo[1,2-a]pyrimidine (Fig. 10, Step 3).

The NMR measurement results of the resulting 6-tributylstannyl-2-(4'-hydroxyphenyl)imidazo[1,2-a]pyrimidine (internal standard: tetramethylsilane) are shown below.

NMR apparatus employed: JNM-ECP-500 (manufactured by Japan Electron Optics Laboratory Co., Ltd. (JEOD))
$^{1}$H-NMR (solvent: chloroform-dl, resonance frequency: 500 MHz): δ 8.41 (s, 1H), 8.23 (s, 1H), 7.80 (d, J = 8.7 Hz, 2H), 7.63 (s, 1H), 6.93 (d, J = 8.7 Hz, 2H), 1.57-1.51 (m, 6H), 1.37-1.23 (m, 6H), 1.16-1.12 (m, 6H), 0.88 (d, J = 7.3 Hz, 9H)

(Example II-2) Synthesis of 6-tributylstannyl-2-(4'-hydroxyphenyl)imidazo[1,2-a]pyrazine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 11, Step 1).

4.66 g (corresponding to 21.6 mmol) of 2-bromo-4'-hydroxyacetophenone and 2.53 g (corresponding to 14.5

mmol) of 5-bromo-2-aminopyrazlne were dissolved in 100 mL of acetonitrile. The resulting solution was refluxed in an oil bath at 110°C for 3.5 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 10 mL of water and 10 mL of methanol. Then, about 20 mL of a saturated sodium hydrogencarbonate solution was added thereto, and the mixture was sonicated for 10 minutes using an ultrasonic washing machine. Precipitates were filtered and recovered from the resulting mixture, sufficiently washed with water, and dried under reduced pressure, to obtain 1.32 g (corresponding to 4.55 mmol) of 6-bromo-2-(4'-hydroxyphenyl)imidazo[1,2-a]pyrazine (Fig. 11, Step 2).

1.00 g (corresponding to 3.45 mmol) of 6-bromo-2-(4'-hydroxyphenyl)imidazo[1,2-a]pyrazine was dissolved in 50.0 mL of dioxane, and 20.0 mL of triethylamine was added thereto. Then, 4.5 mL (corresponding to 5.18 mmol) of bis (tributyltin) and 239 mg (at a catalytic amount) of tetrakis-triphenylphosphine palladium were added thereto. After the reaction mixture was stirred at 90°C for 24 hours, the solvent was distilled off under reduced pressure. The residue was purified by flash silica gel column chromatography (elution solvent: hexane/ethyl acetate = 1/1), to obtain 314 mg (corresponding to 0.628 mmol) of 6-tributylstannyl-2-(4'-hydroxyphenyl)imidazo[1,2-a]pyrazine (Fig. 11, Step 3).

The NMR measurement results of the resulting 6-tributylstannyl-2-(4'-hydroxyphenyl)imidazo[1,2-a]pyrazine (internal standard: tetramethylsilane) are shown below.

NMR apparatus employed: JNM-ECP-500 (manufactured by Japan Electron Optics Laboratory Co., Ltd. (JEOL))

$^1$H-NMR (solvent: chloroform-dl, resonance frequency: 500 MHz): δ 9.21 (s, 1H), 7.95 (s, 1H), 7.79 (d, J = 8.7 Hz, 2H), 7.77 (s, 1H), 6.91 (d, J = 8.7 Hz, 2H), 1.70-1.55 (m, 6H), 1.38-1.31 (m, 6H), 1.18-1.15 (m, 6H), 0.89 (d, J = 7.3 Hz, 9H)

$^{13}$C-NMR (solvent: chloroform-dl, resonance frequency: 125 MHz): δ 157.3, 146.4, 143.5, 140.3 128.0, 124.9, 123.6, 116.1, 106.9, 29.0, 27.3, 13.7, 10.0.

(Example 11-3) Synthesis of 2-(4'-hydroxyphenyl)-6-iodoimidazo[1,2-a]pyrazine

314 mg (corresponding to 0.628 mmol) of 6-tributylstannyl-2- (4'-'hydroxyphenyl)imidazo[1,2-a]pyrazine obtained from Example 11-2 was dissolved in 5.0 mL of dichloromethane, to which 114 mg (corresponding to 0.942 mmol) of iodine dissolved in 5.0 mL of dichloromethane was added. The reaction mixture was stirred at the temperature of 0°C for 10 minutes and at room temperature for 30 hours. Then, a saturated aqueous sodium hydrogencarbonate solution and a saturated aqueous sodium thiosulfate solution were added thereto. Precipitates were filtered and recovered, washed with water and ethyl acetate in this order, and dried under reduced pressure, to obtain 131 mg (corresponding to 0.389 mmol) of 2-(4'-hydroxyphenyl)-6-iodoimdazo[1,2-a]pyrazine (Fig. 12, Step 1).

The NMR measurement results of the resulting 2-(4'-hydroxyphenyl)-6-iodoimidazo[1,2-a]pyrazine (internal standard: tetramethylsilane) are shown below.

NMR apparatus employed: JNM-ECP-500 (manufactured by Japan Electron Optics Laboratory Co., Ltd. (JEOL))

$^1$H-NMR (solvent: dimethylformamide-d7, resonance frequency: 500 MHz): δ 9.89 (s, 1H), 9.01 (s, 1H), 8.82 (s, 1H), 8.42 (s, 1H), 7.92 (d, J = 8.7 Hz, 2H), 6.93 (d, J = 8.7 Hz, 2H).

(Example II-4) Synthesis of 8-tributylstannyl-2-(4'-hydroxyphenyl)imidazo[1,2-a]pyridine

50 mL of ethyl acetate was added to 28.17 g (corresponding to 126 mmol) of cupric bromide to obtain a suspension, to which a solution of 8.18 g (corresponding to 60.0 mmol) of 4'-hydroxyacetophenone in a mixed solution of 50 mL of ethyl acetate and 50 mL of chloroform was added. Then, the resulting mixture was refluxed. After 5 hours, the reaction solution was cooled down to room temperature and filtered. The resulting filtrate was concentrated under reduced pressure. The residue was dissolved in ethyl acetate and subjected to decoloring operation with addition of active charcoal. Then, the resulting solution was filtered and concentrated. The resulting crude product was purified by flash silica gel column chromatography (elution solvent: chloroform/methanol = 20/1), and recrystallized from ethyl acetate/petroleum ether, to obtain 7.25 g (corresponding to 33.7 mmol) of 2-bromo-4'-hydroxyacetophenone (Fig. 13, Step 1).

432 mg (corresponding to 2.01 mmol) of 2-bromo-4'-hydroxyacetophenone and 348 mg (corresponding to 2.01 mmol) of 3-bromo-2-aminopyridine were dissolved in 20 mL of acetonitrile. The resulting solution was refluxed in an oil bath at 110°C for 6 hours. After the completion of the reaction, the reaction solution was cooled down to room temperature, and precipitates were filtered and recovered. The precipitates were washed with acetonitrile and dried under reduced pressure. The resulting crude crystals were suspended in a mixed solution of 8 mL of water and 8 mL of methanol. Then, about 8 mL of a saturated sodium hydrogencarbonate solution was added thereto, and the mixture was sonicated for 5 minutes using an ultrasonic washing machine. Precipitates were filtered and recovered from the resulting mixture, sufficiently washed with water, and dried under reduced pressure, to obtain 368 mg (corresponding to 1.27 mmol) of 8-bromo-2-(4'-hydroxyphenyl)imidazo[1,2-a]pyridine (Fig. 13, Step 2).

75.2 mg (corresponding to 0.260 mmol) of 8-bromo-2-(4'-hydroxyphenyl)imidazo[1,2-a]pyridine was dissolved in 10.0 mL of dioxane, and 2.0 mL of triethylamine was added thereto. The, 0.20 mL (corresponding to 0.39 mmol) of bis(tributyltin) and 20.1 mg (at a catalytic amount) of tetrakis-triphenylphosphine palladium were added thereto. After the reaction mixture was stirred at 90°C for 11 hours, a solvent was distilled, off under reduced pressure. The residue was purified by flash silica gel column chromatography (elution solvent: hexane/ethyl acetate = 1/1), to obtain 62.5 mg (corresponding to 0.125 mmol) of 8-tributylstannyl-2-(4'hydroxyphenyl)imidazo[1-,2-a]pyridine (Fig. 13, Step 3).

The NMR measurement results of the resulting 8-tributylstannyl-2-(4'-hydroxyphnyl)imidazo[1,2-a]pyridine (internal standard: tetramethylsilane) are shown below.

NMR apparatus employed: JNM-ECP-500 (manufactured by Japan Electron Optics Laboratory Co., Ltd. (JEOL)) $^1$H-NMR (solvent: chloroform-dl, resonance frequency: 500 MHz): δ 8.01 (d, J = 6.4 Hz, 1H), 7.87 (d, J = 8.7 Hz, 2H), 7.70 (s, 1H), 7.17 (d, J = 6.4 Hz, 1H), 6.87 (d, J = 8.7 Hz, 2H), 6.68-6.66 (m, 1H), 1.69-1.56 (m, 6H), 1.38-1.30 (m, 6H), 1.28-1.16 (m, 6H), 0.88 (t, J = 7.3 Hz, 9H.)

$^{13}$C-NMR (solvent: chloroform-dl, resonance frequency: 125 MHz) ; δ 145.2, 141.0, 139.2, 132.4, 131.8, 127.7, 127.3, 125.0, 115.4, 112.2, 106.4, 29.2, 27.4, 13.7, 10.2.

(Example 11-5) Synthesis of [$^{123}$I]-2-(4'-hydroxyphenyl)-6-iodoimidazo[1,2-a]pyrimidine

To 100 μL of a solution of 6-tributylstannyl-2-(4'-hydroxyphenyl)imidazo[1,2-a]pyrimidine in methanol (at a concentration of 1 mg/mL), 100 μL of 2 mol/L hydrochloric acid, [$^{123}$I]sodium iodide of 621 MBq (150 μL in volume), 20 μL of 1 mmol/L sodium iodide solution and 20 μL of 10% (w/v) hydrogen peroxide were added. After the mixed solution was heated at 50°C for 10 minutes, the solution was subjected to HPLC under the same conditions as described in Example I-2, to obtain [$^{123}$I]-2-(4'-hydroxyphenyl)-6-iodolmidazo[1,2-a]pyrimidine as a fraction.

The same operation as the preceding paragraph was performed to obtain [$^{123}$I]-2-(4'-hydroxyphenyl)-6-iodoimidazo[1,2-a]pyrimidine (the amount of reagents to be added: 150 μL of a solution of 6-tributylstannyl-2-(4'-hydroxyphenyl)-6-iodoimidazo[1,2-a]pyrimidine in methanol (concentration: 1 mg/mL), 75 μL of 2 mol/L hydrochloric acid, [$^{123}$I]sodium iodide of 487 MBq (150 μL in volume), 20 μL of 1 mmol/L sodium iodide solution and 30 μL of 10% (w/v) hydrogen peroxide).

Two fractions obtained by the operations of the two preceding paragraphs were mixed, and 10 ml of water was added thereto. The resulting solution was passed through a reversed phase column (trade name: Sep-Pak (registered trademark, Waters Investments Limited) Light C8 Cartridges manufactured by Waters: the packed amount of the packing agent: 145 mg) so that the column adsorbs and collects [$^{123}$I]-2-(4'-hydroxyphenyl)-6-iodoioimidazo[1,2-a]pyrimidine. The column was rinsed with 1 mL of water, and then 1 mL of diethylether was passed therethrough to elute [$^{123}$I]-2-(4'-hydroxyphenyl)-6-iodoimidazo[1,2-a]pyridine. The amount of radioactivity of the obtained compound was 67 MBq immediately after the synthesis. Further, the TLC analysis was conducted under the following conditions, and as a result, the radiochemical purity of the compound was 92.5%.

TLC analysis conditions:

TALC plate: Silica Gel 60 $F_{254}$ (trade name; manufactured by Merck & Co., Inc.)
Mobile phase: chloroform/methanol/triethylamine = 100/1/2 Detector: Rita Star (trade name; manufactured by raytest)

(Example II-6) Synthesis of [$^{123}$I]-2-(4'-hydroxyphenyl)-6-iodoimidazo[1,3]pyrazine

To 100 μL of a solution of 6-tributylstannyl-2-(4'-hydroxyphenyl)imidazo[1,2-a]pyrazine in methanol (concentration: 1 mg/mL), 75 μL of 2 mol/L hydrochloric acid, [$^{123}$I]sodium iodide of 469 MBq (100 μL in volume), 20 μL of 1 mmol/L sodium iodide solution and 20 μL of 10% (w/v) hydrogen peroxide were added. After the mixed solution was heated at 50°C for 10 minutes, the solution was subjected to HPLC under the same conditions as described in Example I-2, to obtain [$^{123}$I]-2-(4'-hydroxyphenyl)-6-iodoimidazo[1,3]pyrazine as a fraction.

10 ml of water was added to the fraction. The resulting solution was passed through a reversed phase column (trade name: Sep-Pak (registered trademark, Waters Investments Limited) Light C8 Cartridges manufactured by Waters: the packed amount of the packing agent: 145 mg) so that the column adsorbs and collects [$^{123}$I]-2-(4'-hydroxyphenyl)-6-iodoimidazo[1,2-a]pyrazine. The column was rinsed with 1 mL of water, and then 1 mL of diethyl ether was passed therethrough to elute [$^{123}$I]-2-(4'-hydroxyphenyl)-6-iodoimidazo[1,2-a]pyrazine. The amount of radioactivity of the obtained compound was 133 MBq immediately after the synthesis. Further, the TLC analysis was conducted under the same conditions as described in Example II-5, and as a result, the radiochemical purity of the compound was 99.0%.

(Example II-7) Synthesis of [$^{123}$I]-2-(4'-hydroxyphenyl)-8-iodoimidazo[1,2-a]pyridine

To 70 μL of a solution of 8-tributylstannyl-2-(4'-hydroxyphenyl)imidazo[1,2'-a']pyrazine in methanol (concentration: 1 mg/mL), 50 μL of 2 mol/L hydrochloric acid, [$^{123}$I]sodium iodide of 454 MBq (100 μL in volume), 20 μL of 1 mmol/L sodium iodide solution and 20 μL of 10% (w/v) hydrogen peroxide were added. After the mixed solution was heated at 50°C for 10 minutes, the solution was subjected to HPLC under the same conditions as described in Example I-2, to obtain [$^{123}$I]-2-(4'-hydroxyphenyl)-8-iodoimidazo[1,2-a]pyridine as a fraction.

10 ml of water was added to the traction. The resulting solution was passed through a reversed phase column (trade name: Sep-Pak (registered trademark,Waters Investments Limited) Light C18 Cartridges manufactured by Waters: the packed amount of the packing agent: 130 mg) so that the column adsorbs and collects [$^{123}$I]-2-(4'-hydroxyphenyl)-8-iodoimidazo[1,2-a]pyridine. The column was rinsed with 1 mL of water, and then 1 mL of diethyl ether was passed therethrough to elute [$^{123}$I]-2-(4'-hydroxyphenyl)-8-iodoimidazo[1,2,-a]pyridine. The amount of radioactivity of the obtained compound was 185 MBq immediately after the synthesis. Further, the TLC analysis was conducted under the same conditions as described in Example II-5, and as a result, the radiochemical purity of the compound was 91.7%.

(Reference Example 1) Synthesis of [$^{125}$I]-IMPY

[$^{125}$I]-IMPY was prepared in accordance with the following steps for use in Comparative Example (Comparative Example I-6) for evaluation on $\log P_{octanol}$.

In accordance with the literature (Zhi-Ping Zhuang et al., J. Med. Chem, 2003, 46, p.237-243), 6-tributylstannyl-2-[4'-(N,N-dimethylamino)phenyl]imidaxo[1,2-a]pyridine was synthesized, and dissolved in methanol (concentration: 1mg/mL). To 53 μL of the resulting solution, 75 μL of 1 mol/L hydrochloric acid, 20 μL of [$^{125}$I]sodium iodide of 13.5 MBq, and 10 μL of 10% (w/v) hydrogen peroxide were added. After the mixed solution was left to stand at 50°C for 10 minutes, the solution was subjected to HPLC under the same conditions as described in Example I-2, to obtain [$^{125}$I]-IMPY fraction.

10 ml of water was added to the fraction. The resulting solution was passed through a reversed phase column (trade name: Sep-Pak (registered trademark, Waters Investments Limited) Light C18 Cartridges manufactured by Waters; the packed amount of the packing agent: 130 mg), so that the column adsorbs and collects the [$^{125}$I]-IMPY.
The column was rinsed with 1 mL of water, and then 1 mL of ethanol was passed therethrough, to elute [$^{125}$I]-IMPY. The obtained radioactivity was 2.6 MBq immediately after the synthesis. Further, the TLC analysis was conducted under the same conditions as described in Example 1-2, and as a result, the radiochemical purity of the compound was 98.0%.

(Reference Example 2) Synthesis of [$^{123}$I]-IMPY

[$^{123}$I]-IMPY was prepared in accordance with the following steps for use in Comparative Examples (Comparative Example I-7) for evaluations on accumulation in brain.

In accordance with the literature (Zhi-Ping Zhuang et al., J. Med. Chem, 2003, 46, p.237-243), 6-tributylstannyl-2- [4'-(N,N-dimethylamino)phenyl]imidazo[1,2-a]pyridine was synthesized, and dissolved in methanol (concentration: 1mg/mL). To 53 μL of the resulting solution, 100 μL of 1 mol/L hydrochloric acid, 20-50 μL of [$^{123}$I]sodium iodide of 190-240 MBq, 10 μL of a 1 mmol/L sodium iodide solution and 10 μL of 10% (w/v) hydrogen peroxide were added. After the mixed solution was left to stand at 50°C for 10 minutes, the solution was subjected to HPLC under the same conditions as described in Example I-2, to obtain [$^{123}$I]-IMPY fraction.

10 ml of water was added to the fraction. The resulting solution was passed through a reversed phase column (trade name: Sep-Pak (registered trademark, Waters Investments Limited) Light C18 Cartridges manufactured by Waters; the packed amount of the packing agent: 130 mg), so that the column adsorbs and collects the [$^{123}$I]-IMPY. The column was rinsed with 1 mL of water, and then 1 mL of ethanol was passed therethrough, to elute [$^{123}$I]-IMPY. The obtained radioactivity was 47-56 MBq immediately after the synthesis. Further, the TLC analysis was conducted under the same conditions as described in Example I-2, and as a result, the radiochemical parity of the compound was 98.0%.

(Examples I-11 two I-14, Comparative Examples I-1 to I-5)

Measurement of amyloid affinity

Affinity of the present compounds with amyloid was examined by the following *in vitro* binding tests.

(1) $A\beta_{1-40}$ (Peptide Institute) was dissolved in phosphate buffer (pH 7.4) and shaken at 37°C for 62-72 hours, to obtain a suspension of aggregated Aβ. (concentration: 1 mg/mL equivalent, hereinafter referred to as amyloid suspension in these Examples).

(2) According to the method described in a literature (Naiki, H., et al., Laboratory Investigation 74, p. 374-383

(1996)), the amyloid suspension was subjected to qualitative experiment based on fluorescence spectrophotometric method using Thioflavin T (manufactured by Fluka) to confirm that the aggregated Aβ obtained in (1) was amyloid (measurement conditions: excitation wavelength of 446 nm, and fluorescence wavelength of 490 nm).

(3) According to the method described in a literature (Wang, Y., et al., J. Labeled Compounds Radiopharmaceut. 44, S239 (2001)), [$^{125}$I]2-(3'-iodo-4'-aminophenyl)benzothiazole (hereinafter referred to as [$^{125}$I]3'-I-BTA-0) was prepared from a labeling precursor 2-(4'-aminophenyl)bensothiazole, and dissolved in ethanol.
[$^{125}$I]sodium iodide of 12-71 MBq (10-30 μL in volume) was used for the production, to obtain [$^{125}$I]3'-I-BTA-0 of 1-22 MBq Immediately after the synthesis. As Congo-Red, Thioflavin T and 6-methyl-2-[4'-(N,N-dimethylamino)phenyl]benxothiazole (hereinafter referred to as 6-He-BTA-2), commercially available reagents were weighed and used as they were.

(4) 2-(3'-Iodo-4'-aminophenyl)benzothiazole (hereinafter referred to as 3"-I-BTA-0) and IMPY were synthesized according to the methods described in a literature (Wang. Y., et al., J. Labelled Compounds Radiopharmaceut. 44, S239 (2001)) and a literature (Zhuang, Z. P., et al., J. Med. Chem. 46, 237 (2003)) respectively.

(5) Samples in which [$^{125}$I]3'-I-BTA-0, each compound for evaluation and amyloid were dissolved in a 0.1 % bovine serum albumin-containing phosphate buffer (pH 7.4) at final concentration shown in Table 2 were prepared. The resulting samples were placed in each well (about 0.3 mL in volume) of a 96-well microplate.

Table 2: Final concentrations of each compound in sample solutions

| Experiment | Compound for evaluation | Concentration of compound for evaluation | [$^{125}$I] 3'-I-BTA-0 concentration | Amyloid |
|---|---|---|---|---|
| Comparative Example I-1 | 3'-I-BTA-0 | Each concentration of 0, 0.001 0.01, 0.1 1, 10, 100, 1000 nmol/L | 400 pmol/L | 1 μmol/L |
| Comparative Example I-2 | Congo-Red | | | |
| Comparative Example I-3 | Thioflavin T | | | |
| Comparative Example I-4 | 6-Me-BTA-2 | | | |
| Comparative Example I-5 | IMPY | | | |
| Example I-11 | Compound 1 | | | |
| Example I-12 | Compound 2 | | | |
| Example I-13 | Compound 3 | | | |
| Example I-14 | Compound 4 | | | |

(6) The microplate filled with the sample solutions was shaken at a given rate (400 rpm) at 22°C for 3 hours. Then, each sample solution was filtered through a glass fiber filter (trade name: Mulutiscreen™-FC, manufactured by Millipore), to separate the [$^{125}$I]3'-I-BTA-0- attached to amyloid from the [$^{125}$I]3'-I-BTA-0 free from amyloid.

(7) The glass fiber filter used for the filtration of each sample solution was washed with a 0.1 % bovine serum albumin-containing phosphate buffer (pH 7.4) (0.5 my x 5), and radioactivity of the glass fiber filter was measured with an autowell gamma system (manufactured by Aloka, Type: ARC-301B). The radioactivity was used as the radioactivity level of each sample solution attached to amyloid for calculating an inhibition ratio (hereinafter, A denotes the radioactivity level in a sample with zero (0) concentration of each compound for evaluation, and B denotes the radioactivity level in a sample with 0.001 nmol/L or higher concentration of each compound for evaluation).

(8) Separately, a solution containing 15 μmol/L of 6-Me-BTA-2, 400 μmol/L of [$^{125}$I]'-I-BTA-O and 1 μmol/L of A$\beta_{1-40}$ in a 0.1 % bovine serum albumin-containing phosphate buffer (pH 7.4) was prepared and subjected to the same procedures as described above in (6) and (7) to measure a radioactivity level. The measured radioactivity level was defined as the background radioactivity level, and used in the calculation of the inhibition ratio (hereinafter referred to as BG).

(9) Using the radioactivity levels measured above in (7) and (8), the inhibition ratio was determined by the following formula (1).

$$\text{Inhibition Ratio} = \frac{B - BG}{A - BG} \times 100 \quad (\%) \quad \cdots (1)$$

A graph in which values converted by probit transformation from the obtained inhibition ratios were plotted relative to logarithms of concentrations of compounds for evaluation was prepared to obtain an approximate straight line by the least square method. Using the line, the concentration of each compound for evaluation was determined, at which the radioactivity level is half of the level of the sample free from each compound for evaluation, and was defined as a 50 % inhibition concentration of each compound (hereinafter referred to as $TC_{50\%}$ value). Using the value as an indicator, affinity of each compound for evaluation with amyloid (aggregated $A\beta_{1-40}$) was devaluated.

$IC_{50\%}$ value of each compound for evaluation is shown in Table 3. Compounds 1 to 4 all showed $IC_{50\%}$ values of less 85 than 100 and had higher affinity with amyloid (aggregated $A\beta_{1-40}$) than Congo-Red and Thioflavin T. The results show that Compounds 1 to 4 have good affinity with amyloid (aggregated $A\beta_{1-40}$). In particular, Compound 1 had higher affinity with amyloid (aggregated $A\beta_{1-40}$) than 3'-I-BTA-0 and 6-Me-BTA-2 and had the affinity comparable to IMPY.

Table 3: $IC_{50\%}$ values of the present compounds

| Experiment | Compound for evaluation | $IC_{50\%}$ values (nmol/L) |
|---|---|---|
| Comparative Example I-1 | 3'-I-BTA-0- | 10.1 |
| Comparative Example I-2 | Congo-Red | >1000 |
| Comparative Example I-3 | Thioflavin T | >1000 |
| Comparative Example I-4 | 6-Me-BTA-2 | 25.4 |
| Comparative Example I-5 | IMPY | 4.0 |
| Example I-11 | Compound 1 | 4.4 |
| Example I-12 | Compound 2 | 46.0 |
| Example I-13 | Compound 3 | 54.4 |
| Example I-14 | Compound 4 | 54.1 |

(Example I-15, Examples II-8 to II-10, Comparative Example 1-6) Measurement of partition coefficient based on the octanol extraction method

Partition coefficients based on the octanol extraction method (hereinafter referred to as $\log P_{octanol}$) were measured, which are generally known as an indicator of permeability of compounds through the blood-brain barrier (hereinafter referred to as BBB).

A diethyl ether solution of Compound 5 prepared in Example 1-2 (Example 1-15), a diethyl ether solution of Compound 9 prepared in Example II-5 (Example II-8), a diethyl ether solution of Compound 10 prepared in Example II-6 (Example II-9), a diethyl ether solution of Compound 11 prepared in Example II-7 (Example II-10) and a diethyl ether solution of $[^{123}I]$-IMPY prepared in Reverence Example 1 (Comparative Example I-6) were each diluted with 10 mg/mL ascorbic acid-containing physiological saline solution, and adjusted to radioactive concentration of 20-30 MBq/mL. 10 μL each of the prepared sample solution was respectively added to 2 mL of octanol, further, 2 mL of 10 mmol/L phosphate buffer (pH 7.4) was added, and stirred for 30 seconds. After the mixture was centrifuged with a low-speed centrifuge (2000 rpm x 60 min.), the octanol layer and the water layer were sampled each in an amount of 1 mL, and subjected to measurement of radioactivity count with an autowell gamma system (Type: ARC-301B, manufactured by Aloka). Using the obtained radioactivity count, $\log P_{octanol}$ was calculated in accordance with the equation (2).

$$\log P_{octanol} = \log_{10}\left(\frac{\text{Radioactivity count of octanol layer}}{\text{Radioactivity count of water layer}}\right) \cdots (2)$$

The results are shown in Table 4. Compound 5 showed a $\log P_{octanol}$ value of 1.6, and [$^{125}$I]-IMPY showed a $\log P_{octanol}$ value of 2.1. It is known that compounds permeable to EBB show a $\log P_{octanol}$ value between 1 and 3 (Douglas D. Dischino et al., J. Nucl. Med., (1983), 24, p.1030-1038). Thus, it is implied that both compounds have a BBB permeability comparable to IMPY.

Table 4: $\log P_{octanol}$ value of the present compound

| Experiment | Compound | $\log P_{octanol}$ value |
|---|---|---|
| Comparative Example I-6 | [$^{125}$I]-IMPY | 2.1 |
| Example I-15 | Compound 5 | 1.6 |
| Example II-8 | Compound 9 | 1.7 |
| Example II-9 | Compound 10 | 2.3 |
| Example II-10 | Compound 11 | 3.0 |

(Example I-16, Comparative Example I-7) Measurement of transferability into brain and clearance

Using Compound 6, a time course change of radioactive accumulation in brain of male Wistar rats (7-week old) was measured.

0.05 mL (20-30 MBq/mL in radioactive concentration) of a solution of Compound 6 (Example I-16) in a 10 mg/mL ascorbic acid-containing physiological saline solution and 0.05 mL (20-30 MBq/mL in radioactive concentration) of a solution of [$^{123}$I]-IMPY (Comparative Example I-7) prepared above in Reference Example 2 in a 10 mg/mL ascorbic acid-containing physiological saline solution were infected under thiopental anesthesia into the tail vein of respective Wistar rats (7-week old). The rats were sacrificed by bleeding from abdominal artery, and brains were removed and subjected to measurement of radioactivity (hereinafter referred to was A in this Example) with an autowell gamma system (Type: ARC-301B, manufactured by Aloka) and further subjected to measurement of mass of brains 2, 5, 30 and 60 minutes after the injection. Also, radioactivity (hereinafter referred to as B in this Example) of 0.05 mL of a 1000-fold diluted solution of the injected solution was measured in the same manner as above. Using these measurement results, radioactive accumulation per unit weight of brain (%ID/g) at the respective time points was calculated in accordance with the following formula (3).

Two animals were used for both Example I-16 and Comparative Example I-7 at the respective time points.

$$\%ID/g = \frac{A}{B \times 1000 \times brain\ weight} \times 100 \quad \cdots \quad (3)$$

The results are shown in Table 5. As shown in Table 5, Compound 6 showed a accumulation comparable to $^{123}$I-IMPY at the time point of two minutes after the injection, and then showed a tendency to rapidly clear away in 60 minutes. These results suggest that Compound 6 possesses excellent transferability to brain and rapid clearance from brain like $^{123}$I-IMPY.

Table 5: Radioactive accumulation in brain of Compound 6 after intravenous infection (rats)

| Compound | | Radioactive accumulation per unit weight (%ID/g) | | | |
|---|---|---|---|---|---|
| | | After 2 min. | After 5 min. | After 30 min. | After 60 min. |
| Comparative Example I-7 | $^{123}$I-IMPY | 1.02 | 0.99 | 0.20 | 0.08 |
| Example I-16 | Compound 6 | 0.96 | 0.69 | 0.16 | 0.04 |

(Example I-17) Confirmation of imaging of amyloid in brain

The following experiment was carried out in order to examine whether amyloid in brain can be imaged by the

compound of the present invention.

(1) $A\beta_{1-40}$ (manufactured by Peptide Institute) was dissolved in phosphate buffer (pH 7.4) and shaken at 37°C for 72 hours, to obtain a suspension of aggregated Aβ (Aβ concentration: 1 mg/mL equivalent, hereinafter referred to as amyloid suspension in this Example).

(2) 25 μL (corresponding to 25 μg) of the amyloid suspension was injected into an amygdaloid nucleus on one side of a male Wistar rat (7-week old). As a control, 25 μL of a phosphate buffered physiological saline solution (pH 7,4) was injected into an amygdaloid nucleus on the other side of the rat. The rats were examined 1 day after the injection of the amyloid suspension and the phosphate buffered physiological saline solution (pH 7.4).

(3) Compound 6 was dissolved in a 10 mg/mL ascorbic acid-containing physiological saline solution to obtain a sample solution (32 MBq/mL in radioactivity concentration). This solution was injected into the rat through the tail vein (dosage: 0.5 mL, dosed radioactivity: 16 MBq equivalent).

(4) Brain was removed 60 minutes after the injection to prepare a brain slice of 10 μm in thickness with a microtome (type: CM3050S, manufactured by LEICA). The brain slice was exposed to light on an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imagine by use of a fluorescence microscope (type: TE2000-U model; manufactured by NIKON Corporation; excitation, wavelength: 400-440 nm; detection wavelength: 470 nm). Thus, it was confirmed that amyloid was deposited on the slice (Fig. 9b).

Fig. 9 shows images by autoradiogram and Thioflavin T staining of the brain slice of the rat to which amyloid was injected intracerebrally. As shown in Fig. 9, a marked accumulation of radioactivity was observed in the amygdaloid nucleus on the side to which the amyloid suspension was injected. From the result of Thioflavin T staining in the site where radioactivity accumulated, it was confirmed that amyloid was present in the accumulation site. On the other hand, no significant accumulation of radioactivity was observed in the amygdaloid nucleus on the side to which the physiological saline solution was injected, compared with the other sites.

These results suggest that Compound 6 possesses a property of accumulating on intracerebral amyloid, and a capability of imaging intracerebral amyloid.

(Example I-18 to I-20) Reverse mutation test

In order to examine gene mutagenicity or Compounds 1, 2 and 4, reverse test using *Salmonella typhimurium* TA98 and TA100 (hereinafter referred to as Ames test) was conducted.

The test was conducted without addition of S9mix and with addition of S9mix. Dimethylsulfoxide was used as a negative control, A positive control was 2-(2-furyl)-3-(5-nitro-2-furyl)acrylamide in case S9mix was not added, and 2-aminoanthracene in case S9 mix was added.

The amount of each sample to be added to the test plate was 7 dosages (geometric ratio 4) with the maximum dose being 5000 μg/plate. After a sample to be examined and a strain (TA98 or TA100), or a sample to be examined, S9mix and the strain were mixed together, the mixture was multilayered using soft agar on a medium of a test plate, and then incubated at 37°C for 48 hours. Judgment was made by counting the number of reverse mutation colonies on the plate after the incubation, and when the number of reverse mutation colonies was not less than two times the number in negative control any showed concentration-dependent increase, mutagenicity was determined to be positive.

The results are shown in Table 6. The numbers of reverse mutation colonies of the respective strains in the group treated with Compounds 1, 2 and 4 were less than two times the number in the group treated with the negative control, regardless of addition of S9mix and the addition amount of a sample to be examined. From the aforementioned results, it is judged that Compounds 1, 2 and 4 are negative in the Ames test and have no gene mutagenicity.

Table 6: Results of Ames test

| | Compound | Mutagenicity | | | |
|---|---|---|---|---|---|
| | | Without addition of S9mix | | With addition of S9mix | |
| | | TA98 | TA100 | TA98 | TA100 |
| Example I-18 | Compound 1 | Negative | Negative | Negative | Negative |
| Example I-19 | Compound 2 | Negative | Negative | Negative | Negative |
| Example I-20 | Compound 4 | Negative | Negative | Negative | Negative |

(Example II-11, II-12, Comparative Example II-1)

Measurement of transferability into brain and clearance

Using the Compounds 10 and 11, a time course change of radioactive accumulation in brain of male Wistar rats (7-week old) was measured.

0.05 mL (20-31 MBq/mL is radioactivity concentration) of a solution of Compound 10 (Example II-11), Compound 11 (Example II-12) and [$^{123}$I]-IMPY (Comparative Example II-1) prepared in the above Reference Example 2 respectively in a 10 mg/mL ascorbic acid-containing physiological saline solution were injected under thiopental anesthesia into the tail vein of respective Wistar rats. The rats were sacrificed by bleeding from abdominal artery, and brains were removed and subjected to measurement of mass of brains and further subjected to measurement of radioactivity (hereinafter referred to as A in this Example) with a single channel analyzer (detector type: SP-20 manufactured by OHYO TOKEN KOGYO Co., Ltd.) 2, 5, 30 and 60 minutes after the injection. Also, radioactivity (hereinafter referred to as B in this Example) of the rest of the whole body was measured in the same manner as above. Using these measurement results, radioactive accumulation per unit weight of brain (%ID/g) at the respective time points were calculated in accordance with the following formula (4).

Three animals were used for experiment at the respective time points.

$$\%ID/g = \frac{A}{B \times brain\ weight} \times 100 \quad \cdots \quad (4)$$

The results are shown in Table 7. As shown in Table 7, Compounds 10 and 11 showed a significant radioactive accumulation like $^{123}$I-IMPY at the time point of two minutes after the injection, and then showed a tendency to rapidly clear away in 60 minutes. These results suggest that Compounds 10 and 11 possess excellent transferability to brain and rapid clearance from brain like $^{123}$I-IMPY.

Table 7: Radioactive accumulation in brain of the present compound after intravenous injection (rats)

| Compound | | Radioactive accumulation per unit weight (%ID/g) | | | |
|---|---|---|---|---|---|
| | | After 2 min. | After 5 min. | After 30 min. | After 60 min. |
| Example II-11 | Compound 10 | 0.62 | 0.33 | 0.08 | 0.02 |
| Example II-12 | Compound 11 | 0.65 | 0.43 | 0.09 | 0.03 |
| Comparative Example II-11 | $^{123}$I-IMPY | 1.19 | 0.97 | 0.23 | 0.09 |

(Example II-13) *ex vivo* autoradiogram of Compound 10 using rats of amyloid injected model

(1) $A\beta_{1-42}$ (manufactured by Peptide Institute) was dissolved in phosphate buffer (pH 7.4) and shaken at 37°C for 72 hours, to obtain 1 mg/mL of a suspension of aggregated Aβ (hereinafter referred to as amyloid suspension in this Example).

(2) 2.5 μL (corresponding to 25 μg) of the amyloid suspension was injected into an amygdaloid nucleus on one side of a male Wistar rat (7-weak old). As a control, 2.5 μL of a phosphate buffered physiological saline solution (pH 7.4) was injected into an amygdaloid nucleus on the other side of the rat. The rats were examined 1 day after the injection of the amyloid suspension and the phosphate buffered physiological saline solution (pH 7.4).

(3) Compound 10 was dissolved in a 10 mg/mL ascorbic acid-containing physiological saline solution to obtain a sample solution (31 MBq/mL in radioactivity concentration in the sample solution). This solution was injected under thiopental anesthesia into the rat through the tail vein (dosage: 0.5 mL, dosed radioactivity: 15 MBq equivalent).

(4) Brain was removed 60 minutes after the injection to prepare a brain slice on 10 μm in thickness with a microtome (type: CM3050S, manufactured by LEICA). The brain slice was exposed to light on an imaging plate for 20 hours, and then image analysis was carried out by use of a Bio-imaging Analyzer (type: BAS-2500; manufactured by FUJIFILM Corporation).

(5) After the completion of the image analysis using the Bio-imaging Analyzer, pathological staining with Thioflavin T was carried out to perform imaging by use of a fluorescence microscope (manufactured by NIKON Corporation; type: TE2000-U model; excitation wavelength: 400-440 nm; detection wavelength: 470 nm). Thus, it was confirmed that amyloid was deposited on the slice (Fig. 14b).

Fig. 14 shows images by autoradiogram and Thioflavin T staining of the brain slice of the rat to which amyloid was injected intracerebrally. As shown in Fig. 14, a marked accumulation of radioactivity was observed in the amygdaloid nucleus on the side to which the amyloid suspension was injected. On the other hand, no significant accumulation of radioactivity was observed in the amygdaloid nucleus on the side to which the physiological saline solution was injected, compared with the other sites. On the autoradiogram, little accumulation of radioactivity was observed at sites other than the site to which amyloid was injected. From the result of Thioflavin T staining, it was confirmed that amyloid was present in the site where radioactivity accumulated (Fig. 14b). These results suggest that Compound 10 possesses a property of accumulating on intracerebral amyloid and a capability of imaging intracerebral amyloid.

(Example II-14) *ex vivo* autoradiogram of Compound 11 using rats of amyloid injected model

The same operation as in Example II-13 was conducted except using a solution (radioactive concentration of 30 MBq/mL in a sample solution) of Compound 11 in a 10 mg/mL ascorbic acid solution as a sample solution.

Fig. 15 shows images by autoradiogram and Thioflavin T staining of the brain slice of the rant to which amyloid was injected intracerebrally. As shown in Fig. 15, a marked accumulation of radioactivity was observed in the amygdaloid nucleus on the side to which the amyloid suspension was injected. From the result of Thioflavin T staining, it was confirmed that amyloid was present in the site where radioactivity accumulated (Fig. 15.b). On the other hand, no significant accumulation of radioactivity was observed in the amygdaloid nucleus on the side to which the physiological saline solution was injected, compared with the other sites. These results suggest that Compound 11 possesses a property of accumulating on intracerebral amyloid, and a capability of imaging intracerebral amyloid.

(Example II-15) Reverse mutation test

In order to examine gene mutagenicity of Compound 8, reverse mutation test using *Salmomella typhimurium* TA98 and TA100 (hereinafter referred to as Ames test) was conducted.

The test was conducted without addition of S9mix and with addition or S9mix. Dimethylsulfoxide was used as a negative control. A positive control was 2-(2-furyl)-3-(5-nitro-2-furyl)acrylamide in case S9mix was not added, and 2-aminoanthracene in case S9mix was added.

The amount of Compound 8 to be added to the test plate was 7 dosages (geometric ratio 3) with the maximum dose being 5000 μg/plate. After Compound 8 and a strain (TA98 or TA100), or Compound 8, S9mix and the strain were mixed together, the mixture was multilayered using soft agar on a medium of a test plate, and then incubated at 37°C for 48 hours. Judgment was made by counting the number of reverse mutation colonies on the plate after the incubation, and when the number of reverse mutation colonies was not less than two times the number in negative control and showed concentration-dependent increase, mutagenicity was determined to be positive.

The results are shown in Table 8. The numbers of reverse mutation colonies of the respective strains in the group treated with Compound 8 were less than two times the number in the group treated with the negative control, regardless of addition of S9mix and the addition amount of a sample to be examined. On the other hand, a marked increase in the number of reverse mutation colonies was observed in the group treated with the positive control. From the aforementioned results, it is judged that Compound 8 is negative in the Ames test and has no gene mutagenicity.

Table 8: Results of Ames test

| | Compound | Mutagenicity | | | |
|---|---|---|---|---|---|
| | | Without addition of S9mix | | With addition of S9mix | |
| | | TA98 | TA100 | TA98 | TA100 |
| Example II-15 | Compound 8 | Negative | Negative | Negative | Negative |

(Example III-1 to Example III-2) Confirmation of binding to amyloid

In order to study a binding mechanism of a compound of the present invention, an experiment for inhibition of binding to Thioflavin T was conducted using amyloid derived from amylin as a precursor compound. Amylin is an amyloid accumulating in pancreas of type II diabetes.

Method

(1) Amylin (human) (manufactured by Wako) was dissolved in phosphate buffer (pH 7.4) and shaken at 37°C for 72 hours, to obtain a 1 mg/mL suspension of aggregated amylin (hereinafter referred to as amyloid suspension in this Example).
(2) According to the method described in a literature (Naiki, H., et al., Laboratory Investigation 74, p.374-383 (1996)), the amyloid suspension was subjected to qualitative experiment based on fluorescence spectrophotometric method using Thioflavin T (manufactured by Fluka) to confirm that the aggregated amylin obtained in (1) was amyloid (measurement conditions: excitation wavelength of 446 nm, and fluorescence wavelength of 490 nm).
(3) The amyloid suspension was dissolved in 50mM phosphate buffer (pH 7.4) at a concentration of amylin of 15 μM. Also, Thioflavin T was dissolved at a concentration of 15 μM in 50 mM glycin-NaOH buffer (pH
(4) Samples in which each compound for evaluation and amyloid were dissolved in a 50 mM phosphate buffer (pH 7.4) at final concentrations shown in Table 9 were prepared. 50 μL of each amyloid solution and Thioflavin T solution prepared in (3), and 50 μL of a sample solution were placed in each well (about 0.3 mL in volume) of a 96-well microplate.
Table 9: Final concentrations of each compound in sample solutions

| Experiment | compound for evaluation | Concentration of compound for evaluation | Thioflavin T concentration | Amyloid |
|---|---|---|---|---|
| Example III-1 | Compound 1 | Each concentration of 0, 1.5, 15, 30 μmol/ | 5 μmol/L | 2.6 μmol/L |
| Example III-2 | Compound 2 | | | |

(5) A sample in which 100 μL of 50 mM phosphate buffer (pH 7.4) and 50 μL of 50 mM glycin-NaOH buffer (pH 8.5) were mixed was made as a blank, and subjected to the same procedure as in (4) and used for a calculation of inhibition ratio (hereinafter referred to as BG).
(6) The microplate filled with the sample solutions was left fluorescence to stand at room temperature for 30 hours. Then, fluorescence strength of each sample solution was measured (measurement conditions: excitation wavelength of 446 nm, emission wavelength 490 nm) ,with microplate reader (type: SPECTRA, MAX GEMINI XS, manufactured by Devices) (hereinafter, A denotes the fluorescence strength in a sample with zero (0) concentration of each compound for evaluation, and B denotes the fluorescence strength in a saple with 1.5 μmol/L of higher concentration of each compound for evaluation .
(7) Using the fluorescence strength mesuared above in (6), the following formula (5) :.
$$\text{Inhibition Ratio} = \frac{B-BG}{A-BG}\times 100 \quad (\%) \quad \cdots (5)$$

was used to determine the inhibition ratio.
Inhibition ratio of each compound for evaluation is shown in Table 10. Compounds 1 and 2 both prevented a binding of Thioflavin T at any concentration. The results showed that Compounds 1 and 2 competitively inhibit a binding of Thioflavin T. It is generally known that Thioflavin T binds with recognition of β-sheet structure of amyloid. Thus, it has been suggested that Compounds 1 and 2 has a same mechanism of binding to amyloid as Thioflavin T, namely, binds thereto with recognition of β-sheet structure.
Table 10: Inhibition ratio (%) of binding of amyloid (amylin) to Thioflavin T by the present compounds

| Experiment | Compound for evaluation | Inhibition ratio (%) | | |
|---|---|---|---|---|
| | | Concentration of compound for evaluation 1.5 μmol/L | Concentration of compound for evaluation 15 μmol/L | Concentration of compound for evaluation 30 μmol/L |
| Example III-1 | Compound 1 | 8.7 | 41.3 | 27.0 |

(continued)

| Experiment | Compound for evaluation | Inhibition ratio (%) | | |
|---|---|---|---|---|
| | | Concentration of compound for evaluation 1.5 μmol/L | Concentration of compound for evaluation 15 μmol/L | Concentration of compound for evaluation 30 μmol/L |
| Example III-2 | Compound 2 | 11.2 | 16.3 | 32.0 |

(Example III-3) Measurement of amyloid affinity

Affinity of the present compounds with amyloid was examined by the following *in vitro* binding tests.

Method

(1) Using methods described in Examples III-1 and III-2, a 1 mg/mL suspension of aggregated amylin (hereinafter referred to as amyloid suspension in this Example) was prepared.

Insulin with cross β-sheet structure was prepared by the following procedures (2) and (3) which was the method described in the known literature (Burke, M.J. et al., Biochemistry. 11, p. 2435-2439 (1972)) to which a little modification was made.

(2) 5 mg of insulin (manufactured by Sigma Aldrich Japan) was dissolved in 1 mL of deionized water which was adjusted to pH 2 with hydrochloric acid, and the reaction mixture was heated at 90°C for 10 minutes, and then quickly cooled in dry ice/ethanol.

(3) A sample solution of (2) was heated at 90°C for 5 minutes, and then quickly cooled in dry ice/ethanol. After repeating the same procedure for 10 times, change of a sample solution into gel form was visually confirmed.

(4) A sample of (3) was centrifuged (16000g X 15 min.), and then supernatant was removed, precipitates were dissolved with 1 mL of deionized water to obtain a suspension of aggregated insulin (hereinafter referred to as insulin amyloid suspension in this Example).

β2-microglobulin with cross β-sheet structure was prepared by the following procedures (5) and (6) which was the method described in the known literature (Ohhashi, Y. et al., Journal of Biological Chemistry. 280, p. 32843-32848 (2005)) to which a little modification was made.

(5) 50 mM glycin-HCl buffer (pH 2.5) containing 100 mM NaCl buffer was added to a 1 mg/mL solution of β2-microglobulin (manufactured by oriental Yeast Co., ltd.), and then was subjected to ultrasonic treatment at 37 °C for 1 minute in an ultrasonic hot water tank, followed by heating at 37°C for 9 minutes without ultrasonic treatment.

(6) After repeating the procedures of (5) for 17 times, the sample solution was subjected to the same treatment as above in (2). The sample solution was centrifuged (16000g X 15 min.), and then supernatant was removed, and precipitates were dissolved in 0.5 mL of 50 mM glycin-HCl buffer (pH 2.5) containing 100 mM NaCl to obtain a suspension of aggregated β2-microglobulin (hereinafter referred to as β2-m-amyloid suspension in this Example).

(7) According to qualitative experiment based on fluorescence spectrophotometric method using Thioflavin T (manufactured by Fluka) described in Example III-1 and Example III-2, it was confirmed that the aggregated insulin and aggregated β2-microglobulin obtained in (4) and (6) were amyloid.

(7) A solution of Compound 6 which was synthesized by the method above in Example I-3 was prepared (500 LvlBq/mL in radioactivity), and diluted with a 0.1 % bovine serum albumin-containing phosphate buffer (pH 7.4) to prepare a solution of 1.0-101.0 pM which corresponded to the total amount of 2-(4'-hydroxyphenyl)-6-iodoimidazo[1,2-a]pyrimidine.

(8) To each well of 96-well microplate, 50 μL of a solution prepared above in (7) (final concentration of 0.2-20.2 pM) and 50 μL of a solution in which a amylin amyloid suspension, insulin amyloid suspension or β2-m-amyloid suspension was diluted with 0.1 % bovine serum albumin-containing phosphate buffer (pH 7.4) (final concentration of 0.8 μg/mL) were added, and then 150 μL of the same buffer was added.

(9) The microplate was shaken at a given rate (400 rpm) at 22°C for 3 hours. Then, a mixture of each well was filtered through a glass fiber filter (trade name: Mulutiscreen™-FC, manufactured by Milllpore), to separate the Compound 6 attached to amyloid from the free Compound 6.

(10) The glass fiber filter used for the filtration of the mixture was washed with a 0.1 % bovine serum albumin-containing phosphate buffer (0.2 mL x 5), and radioactivity of the glass fiber filter was measured with an autowell gamma system (manufactured by Aloka, Type: ARC-7001).

(11) Relation of the amount of Compound 6 bound to amyloid and the added amount of amyloid were evaluated from the measurement results of (10). Non-specific binding was determined from a sample to which no amyloid suspension was added above in (8) (Example 1-3).

Relation of a concentration of Compound 6 in the sample solution and a radioactive count (CPM) on the glass fiber filter measured above in (11) was shown in Fig. 16. In the group with addition of amyloid suspension (Fig. 16, a group with addition of amylin amyloid, a group with addition of insulin amyloid and a group with addition of β2-m-amyloid), radioactivity was all high compared to the group without addition of amyloid suspension (Fig. 16, a group without addition of amyloid), and radioactivity of the glass fiber filter was increased proportional to the addition concentration of Compound 1. In the conditions of this experiment, all amyloids (and amyloid to which Compound 6 was attached) were lager than the pore size of the glass fiber. Thus, amyloid was supported on the glass fiber, and the radioactive count of the glass fiber became a value reflecting the amount of Compound 6 attached to amyloid. Since the radioactive count of the glass fiber was increased with increase in concentration of Compound 6 and the radioactivity thereof was high compared to the group without addition of amyloid, it was shown that Compound 6 was a compound having a property of specifically binding to amyloid.

(Example IV) Measurement of radioactive distribution ratio in each organ

In order to confirm that a compound of the present invention can be distributed to a target organ and has good clearance to the outside of the body. Compound 6 was used to measure a time-course change of radioactive accumulation to each organ in SD rat (8 week).

To 400 μL of a solution of 6-tributylstannyl-2-(4'-hydroxyphenyl)imidazo[1,2-a]pyridine in acetonitrile (concentration: 1 mg/mL), 400 μL of 1 mol/L sulfuric acid, 10 μL of 1 mmol/L sodium iodide, 270 μL of [$^{123}$I] sodium iodide of 1074 MBq, and 10 μL of 30% (w/v) hydrogen peroxide were added. After the mixed solution was left to stand at 40°C for 10 minutes, the solution was subjected to HPLC under the following conditions, to obtain [$^{123}$I]-2-(4'-hydroxyphenyl)-6-iodoimidazo[1,2-a]pyridine as a fraction.

HPLC conditions:

Column: YMC-Pack Pro C8 (trade name; manufactured by YMC;
size; 4.6 x 150 mm)
Mobile phase: 10 mM formic acid (pH 3.0)/acetonitrile = 80/20 to 80/20 to 10/90 (0 minute to 20 minutes to 30 minutes)
Flow rate: 1.0 mL/min.
Detector: Ultraviolet visible absorptiometer (Detection wavelength: 254 nm) and radioactivity counter
(manufactured by raytest: type STEFFI)

10 ml of water was added to the fraction. The resulting solution was passed through a Sep-Pak C18 column (trade name: Sep-Pak (registered trademark) Light C18 Cartridges manufactured by Waters: the packed amount of the packing agent: 130 mg) so that the column adsorbs and collects [$^{123}$I]-2-(4'-hydroxyphenyl)-6-iodoimidazo[1,2-a]pyridine. The column was rinsed with 1 mL of water, and then 1 mL of diethyl ether was passed therethrough to elute [$^{123}$I]-2-(4'-hydroxyphenyl)-6-iodoimidazo[1,2-a]pyridine (Compound 6). The amount of radioactivity of the obtained compound was 470 MBq at the end of the synthesis. Further, the TLC analysis was conducted under the following conditions, and as a result, the radiochemical purity of the compound was 98%.

TALC analysis conditions:

TLC plate: Silica Gel 60 $F_{254}$ (trade name; manufactured by Merck & Co., Inc.)
Mobile phase: ethyl acetate/methanol/diethylamine = 100/4/1
Detector: Pita Star (trade name; manufactured by raytest)

A solution of Compound 6 in diethylether was diluted with a 10 mg/mL ascorbic acid-containing physiological saline solution and adjusted to 8-12 MBq/mL in radioactive concentration. 0.2 mL each of the prepared sample solution was administered under non-anesthesia into the tail vein of the above rant. The rats were sacrificed by bleeding from abdominal artery, and each organ shown in Table 11 was removed 5, 30, 60 and 180 minutes after the administration. Each removed organ was subjected to measurement of mass and radioactivity with the same method as in Example II-11. Also, radioactivity of the whole body of the rat after removal of the organs (hereinafter referred to as rest of the whole body) was measured. Using these measurement results, radioactive distribution per unit weight of each organ (%ID/g) at the respective time points was calculated in accordance with the following formula (6).
Three animals were used for the experiment at the respective time points.

$$\%ID/g = \frac{R^T}{(R^S + R^R) \times M^T} \times 100 \cdots (6)$$

$R^T$: Radioactivity of target organ (cpm)
$R^S$: Sum of radioactivity of all the organs (cpm)
$R^R$: Radioactivity of the rest of the whole body (cpm)
$M^T$: Mass of target organ (g)

The results are shown in Table 11. As shown in Table 11, Compound 6 was distributed to each organ at the time point of 5 minutes after the administration, and then most of the radioactivity was distributed to small intestine and large intestine. In addition, radioactive distribution thereof was transferred from small intestine to large intestine. Thus, Compound 6 was found to be biliary-excreted rapidly after the administration and have a good clearance to the outside of the body.

Also, observing brain, hearty lung, pancreas and bone in which amyloid is considered to accumulate, obvious radioactive accumulation was found in all these organs at the time point of 5 minutes after the administration, and thus distribution of Compound 6 was confirmed. Moreover, the ratio of the time point of 5 minutes after the administration to the time point of 180 minutes after the administration ((%ID/g at the time point of 5 minutes after the administration)/ (%ID/g at the time point of 180 minutes after the administration)) showed high values such as 122 for brain, 13 for heart, 7 for lung, 14 for pancreas and 4 for bone. Thus, it has been shown that rapid radioactive distribution and rapid clearance are performed in organs in which amyloid ins considered to accumulate.

From the above, it has been shown that a radioactive distribution at an early stage after administration and a rapid clearance to the outside of the body, which are required for an amyloid detecting reagent in a biological tissue, have been attained.

Table 11

| Tissue/ organ | 5 min. after administration | | 30 min. after administration | | 60 min. after administration | | 180 min. after administration | |
|---|---|---|---|---|---|---|---|---|
| | Average | Standard deviation | Average | Standard deviation | Average | Standard deviation | Average | Standard deviation |
| Blood | 0.285 | 0.021 | 0.174 | 0.026 | 0.122 | 0.012 | 0.109 | 0.013 |
| Brain | 0.721 | 0.072 | 0.124 | 0.010 | 0.030 | 0.002 | 0.006 | 0.001 |
| Heart | 0.619 | 0.054 | 0.147 | 0.021 | 0.063 | 0.004 | 0.047 | 0.004 |
| Lung | 0.657 | 0.037 | 0.209 | 0.025 | 0.120 | 0.009 | 0.089 | 0.007 |
| Liver | 2.008 | 0.235 | 0.509 | 0.096 | 0.181 | 0.010 | 0.083 | 0.008 |
| Spleen | 0.462 | 0.024 | 0.140 | 0.028 | 0.075 | 0.003 | 0.072 | 0.016 |
| Pancreas | 0.846 | 0.119 | 0.207 | 0.028 | 0.084 | 0.004 | 0.060 | 0.002 |
| Stomach | 0.360 | 0.079 | 1.025 | 0.164 | 1.043 | 0.227 | 1.081 | 0.373 |
| Small intestine | 1.078 | 0.337 | 6.335 | 0.349 | 8.207 | 0.455 | 1.408 | 0.107 |
| Large intestine | 0.116 | 0.014 | 0.070 | 0.010 | 0.066 | 0.005 | 7.296 | 0.454 |
| Kidney | 0.999 | 0.133 | 0.540 | 0.103 | 0.244 | 0.010 | 0.108 | 0.011 |
| Adrenal gland | 4.648 | 1.354 | 0.642 | 0.226 | 0.260 | 0.064 | 0.020 | 0.035 |
| Bone | 0.222 | 0.017 | 0.088 | 0.015 | 0.058 | 0.002 | 0.053 | 0.010 |
| Marrow | 0.713 | 0.178 | 0.194 | 0.043 | 0.000 | 0.000 | 0.030 | 0.053 |
| Muscle | 0.381 | 0.050 | 0.078 | 0.016 | 0.034 | 0.002 | 0.022 | 0.001 |

INDUSTRIAL APPLICABILITY

The reagent for detecting amyloid in biological tissues according to the present invention can be utilized as diagnostic agents for amyloid protein *in vitro* and *in vivo* in amyloidosis such as systemic amyloidosis.

BREIF DESCRIPTION OF THE DRAWINGS

Fig. 1 is a scheme of synthesis of 6-tributylstannyl-2-(4'-hydroxyphenyl)imidazo[1,2-a]pyridine.
Fig. 2 is a scheme of synthesis of 6-bromo-2-(4'-hydroxyphenyl)imidazo[1,2-a]pyridine.
Fig. 3 is a scheme of synthesis of 2-(4'-hydroxyphenyl)-6-iodoimidazo[1,2-a]pyridine.
Fig. 4 is a scheme of synthesis of 2-(4'-hydroxyphenyl)-6-iodoimidazo[1,2-a]pyrimidine.
Fig. 5 is a scheme of synthesis of 6-(3'-fluoropropoxy)-2-(4'-hydroxyphenyl)imidazo[1,2-a]pyridine.
Fig. 6 is a scheme of synthesis of 6-bromo-2-(4'-hydroxyphenyl)imidazo[1,2-a]pyrimidine.
Fig. 7 is a scheme of synthesis of 6-fluoro-2-(4'-hydroxyphenyl)imidazo[1,2-a]pyridine.
Fig. 8 is a scheme of synthesis of 2-(4'-hydroxyphenyl)-6-nitroimidazo[1,2-a]pyridine.
Fig. 9(a) is an autoradiogram of the brain slice after the injection of Compound 6, and Fig. 9(b) is a fluorescent microscopic image of the Thioflavin T stained sample (a magnification of the site to which the amyloid suspension was injected).
Fig. 10 is a scheme of synthesis of 6-tributylstannyl (4'-hydroxyphenyl)imidazo[1,2-a]pyrimidine.
Fig. 11 is a scheme of synthesis of 6-tribitylstannyl-2-(4'-hydroxyphenyl)imidazo[1,2-a]pyrazine.
Fig. 12 is a scheme of synthesis of 2-(4'-hydroxyphenyl)-6-iodoimidazo[1,2-a]pyrazine.
Fig. 13 is a scheme of synthesis of 8-tributylstannyl-2-(4'-hydroxyphenyl)imidazo[1,2-a]pyridine.
Fig. 14(a) is an autoradiogram of the brain slice after the infection of Compound 60, and Fig. 14(b) is a fluorescent microscopic image of the Thioflavin T stained sample (a magnification of the site to which the amyloid suspension was injected).
Fig. 15(a) is an autoradiogram of the brain slice after the injection of Compound 61, and Fig. 15(b) is a fluorescent microscopic image of the Thioflavin T stained sample (a magnification of the site to which the amyloid suspension was injected).
Fig. 16 is a drawing showing an ability of binding to amyloid of Compound 6.

## Claims

**1.** A reagent for detecting amyloid deposited in a biological tissue, which comprises a compound represented by the following formula (1), or a salt thereof:

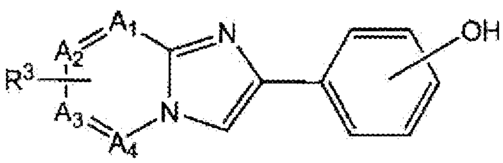

(1)

wherein $A^1$, $A^2$, $A^3$ and $A^4$ independently represent a carbon or a nitrogen, and
$R^3$ is a group represented by the following formula:

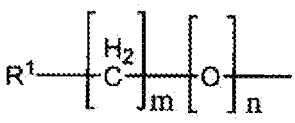

wherein $R^1$ is a radioactive halogen substituent;

m is an integer of 0 to 4; and
n is an integer of 0 or 1,
provided that at least one of $A^1$, $A^2$ $A^3$ and $A^4$ represents a carbon, and $R^3$ binds to a carbon represented by $A^1$, $A^2$, $A^3$ or $A^4$.

2. The reagent according to claim 1, wherein at least three of $A^1$, $A^2$, $A^3$ and $A^4$ represent carbons.

3. The reagent according to claim 2, wherein all of $A^1$, $A^2$, $A^3$ and $A^4$ represent carbons.

4. The reagent according to any one of claims 1 to 3, wherein $R^1$ is selected from the group consisting of $^{18}F$, $^{75}Br$, $^{76}Br$, $^{123}I$, $^{124}I$, $^{125}I$ and $^{131}I$.

5. The reagent according to any one of claims 1 to 4, wherein a biological tissue is brain, heart, lung, pancreas, bone, or joints.

6. A process for production of a radioactive halogen-labeled organic compound, which comprises a step of preparing a reaction solution containing, together with a radioactive halogen ion, a compound represented by the following formula (2) or a salt thereof:

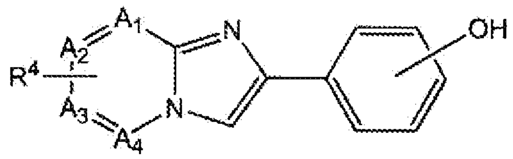

(2)

wherein $A^1$, $A^2$, $A^3$ and $A^4$ independently represent a carbon or a nitrogen, and
$R^4$ is a group represented by the following formula:

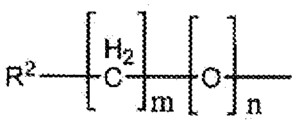

wherein m is an integer of 0 to 4;
n is an integer of 0 or 1; g and
when m = n = 0, $R^2$ is a non-radioactive halogen substituent, nitro subsituent, trialkylammonium substituent having 3 to 12 carbon atoms, trialkylstannyl substituent having 3 to 12 carbon atoms or triphenylstannyl substituent, and when $m \neq 0$ and/or $n \neq 0$, $R^2$ is a non-radioactive halogen substituent, methanesulfonyloxy substituent, trifluoxomethanesulfonyloxy substituent or aromatic sulfonyloxy substituent,
provided that at least one of $A^1$, $A^2$, $A^3$ and $A^4$ represents a carbon, and $R^4$ binds to a carbon represented by $A^1$, $A^2$, $A^3$ or $A^4$; and
a step of giving a reaction condition to the above reaction solution to synthesize a compound represented by the following formula:

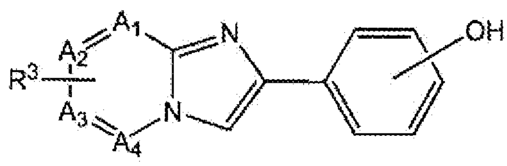

(1)

wherein $A^1$, $A^2$, $A^3$ and $A^4$ are the same as in the formula (2), and
$R^3$ is a group represented by the following formula:

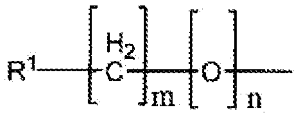

wherein $R^1$ is a radioactive halogen substituent; and
m and n are the same as in the formula (2),
provided that at least one of $A^1$, $A^2$ $A^3$ and $A^4$ represents a carbon, and $R^3$ binds to a carbon represented by $A^1$, $A^2$, $A^3$ or $A^4$.

**7.** The process for production of a radioactive halogen-labeled organic compound, according to claim 6, wherein at least three of $A^1$, $A^2$, $A^3$ and $A^4$ represent carbons.

**8.** The process for production of a radioactive halogen-labeled organic compound, according to claim 7, wherein all of $A^1$, $A^2$, $A^3$ and $A^4$ represent carbons.

**9.** The process for production of a radioactive halogen-labeled organic compound, according to any one of claims 6 to 8, wherein $R^2$ is selected from the group consisting of iodine, bromine, trialkylstannyl substituent having 3 to 12 carbon atoms and triphenylstannyl substituent, the radioactive halogen ion is selected from the group consisting of $^{123}I$ ion, $^{125}I$ ion, $^{125}I$ ion and $^{131}I$ ion, and $R^1$ is selected from the group consisting of $^{123}I$, $^{124}I$, $^{125}I$ and $^{131}I$.

**10.** The process for production of a radioactive halogen-labeled organic compound, according claim 9, wherein $R^2$ is selected from the group consisting of an iodine, trimethylstannyl substituent, tributylstannyl substituent and triphenylstannyl substituent.

**11.** The process for production of a radioactive halogen-labeled organic compound, according to any one of claims 6 to 8, wherein $R^1$ is selected from the group consisting of a nitro substituent, trialkylammonium substituent having 3 to 12 carbon atoms, methanesulfonyloxy substituent, trifluoromethanesulfonyloxy substituent and aromatic sulfonyloxy substituent, the radioactive halogen ion is $^{18}F$ ion, and $R^1$ is $^{18}F$.

**12.** The process for production of a radioactive halogen-labeled organic compound, according to claim 11, wherein $R^2$ is trifluoromethanesulfonyloxy substituent or toluene sulfonyloxy substituent.

**13.** The process for production of a radioactive halogen-labelled organic compound, according to any one of claims 6 to 8, wherein $R^2$ is a bromine, the radioactive halogen ion is $^{75}Br$ ion or $^{76}Br$ ion, and $R^1$ is $^{75}Br$ or $^{76}Br$.

**14.** The precursor compound for preparing a radioactive halogen-labeled organic compound, which is represented by the following formula (1), or a salt thereof:

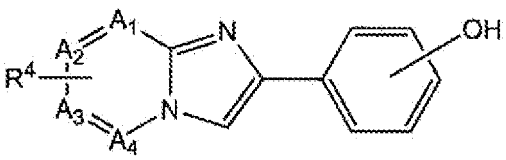

(2)

wherein $A^1$, $A^2$, $A^3$ and $A^4$ independently represent a carbon or a nitrogen, and
$R^4$ is a group represented by the following formula:

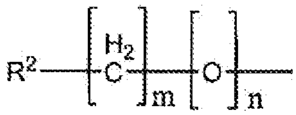

wherein m is an integer of 0 to 4;
n is an integer of 0 or 1; and
when m = n = 0, $R^2$ is a non-radioactive halogen substituent, nitro subsituent, trialkylammonium substituent having 3 to 12 carbon atoms, trialkylstannyl substituent having 3 to 12 carbon atoms or triphenylstannyl substituent, and when $m \neq 0$ and/or $n \neq 0$, $R^2$ is a non-radioactive halogen substituent, methanesulfonyloxy substituent, trifluoromethanesulfonyloxy substituent or aromatic sulfonyloxy substituent,
provided that at least one of $A^1$, $A^2$, $A^3$ and $A^4$ represents a carbon, and $R^4$ binds to a carbon represented by $A^1$, $A^2$, $A^3$ or $A^4$.

**15.** The precursor compound for preparing a radioactive halogen-labeled organic compound, or a salt thereof, according to claim 14, wherein at least three of $A^1$, $A^2$, $A^3$ and $A^4$ represent carbons.

**16.** The precursor compound for preparing a radioactive halogen-labeled organic compound, or a salt thereof, according to claim 15, wherein all of $A^1$, $A^2$, $A^3$ and $A^4$ represent carbons.

**17.** The precursor compound for preparing a radioactive halogen-labeled organic compound, or a salt thereof, according to any one of claims 14 to 16, wherein $R^2$ is selected from the group consisting of an iodine, bromine, trimethylstannyl substituent, tributylstannyl substituent, trifluoromethanesulfonyloxy substituent and triphenylstannyl substituent.

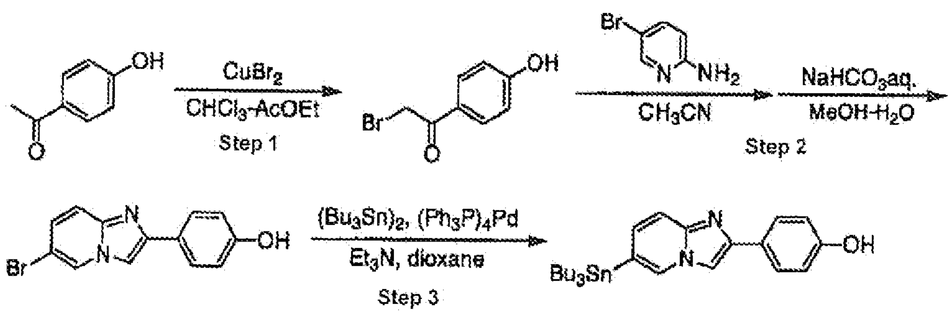


Figure 1

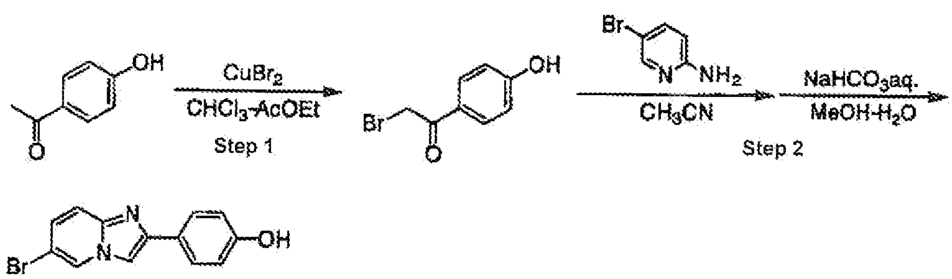


Figure 2

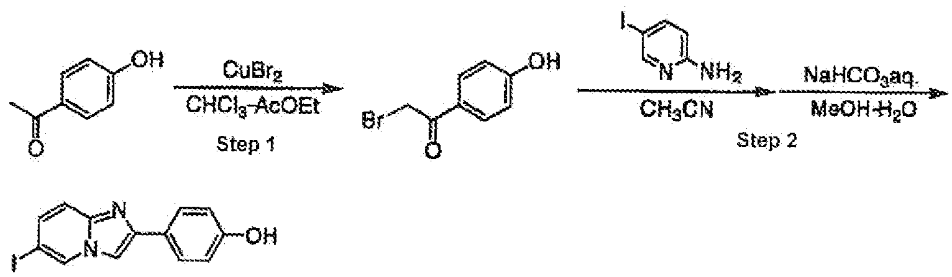


Figure 3

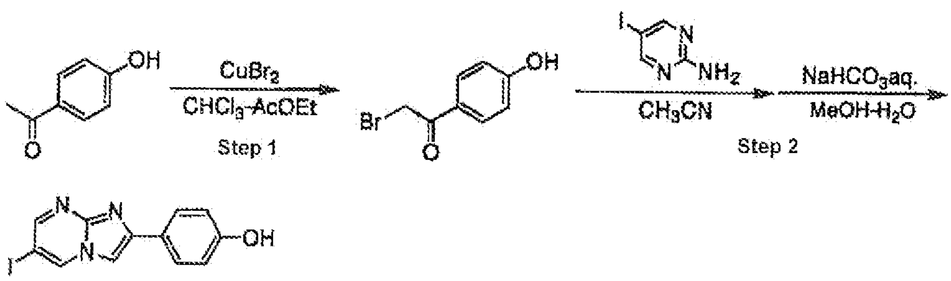


Figure 4

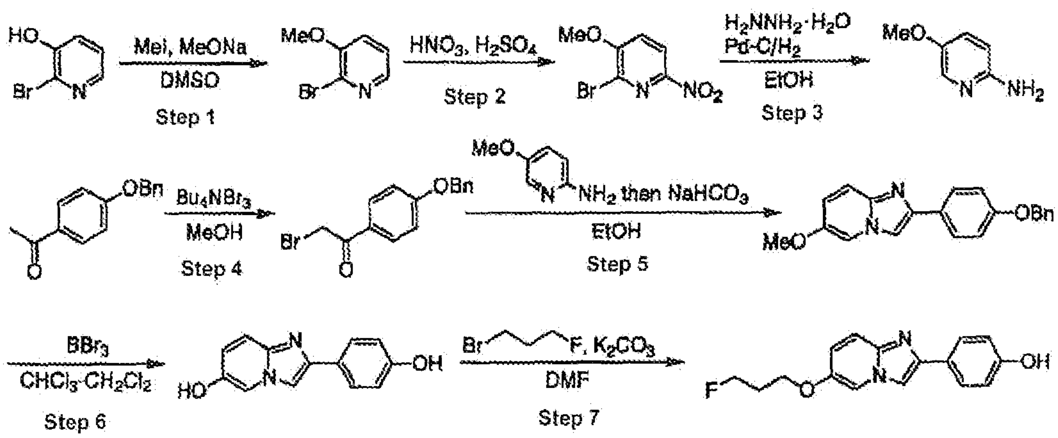


Figure 5

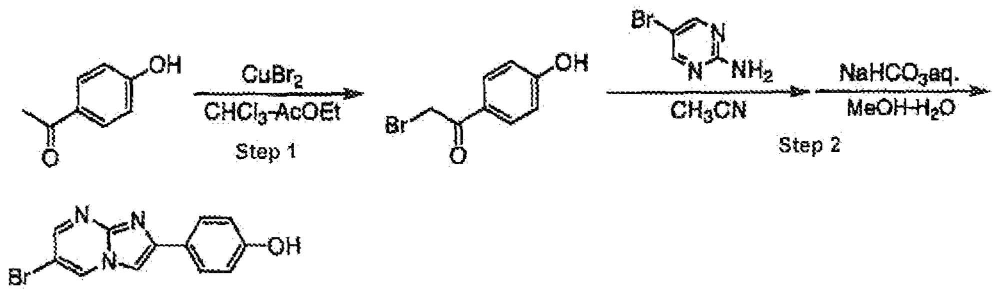


Figure 6

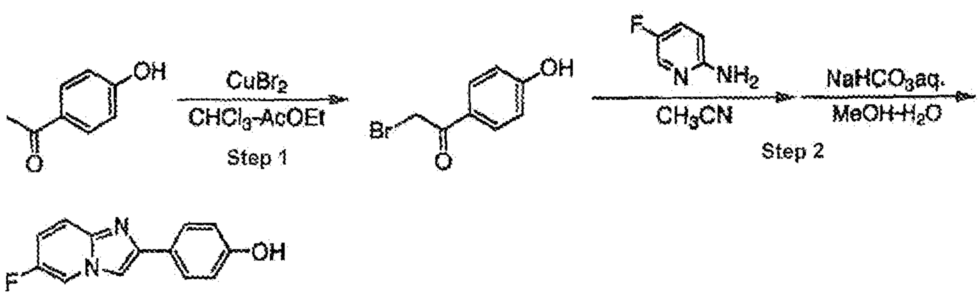


Figure 7

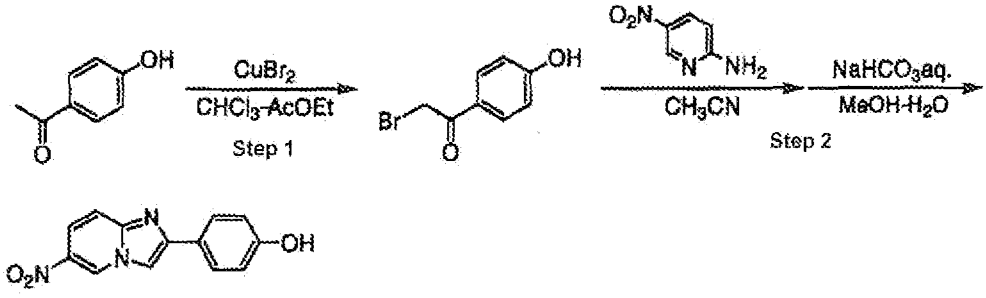


Figure 8

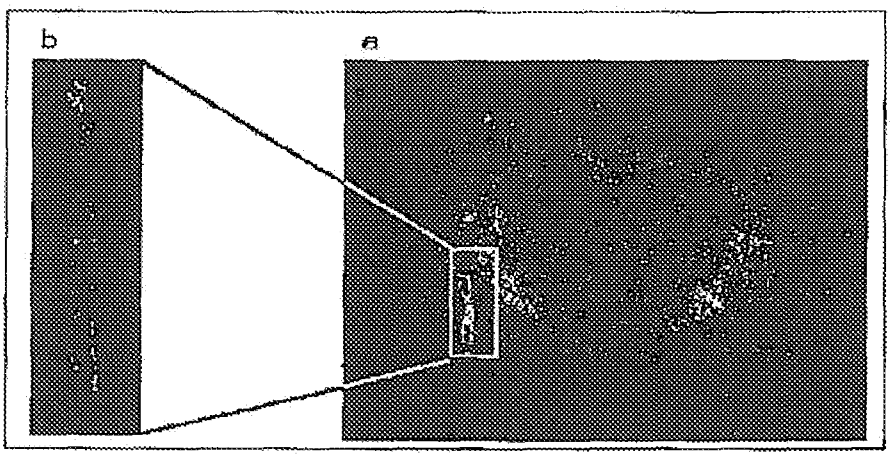


Figure 9

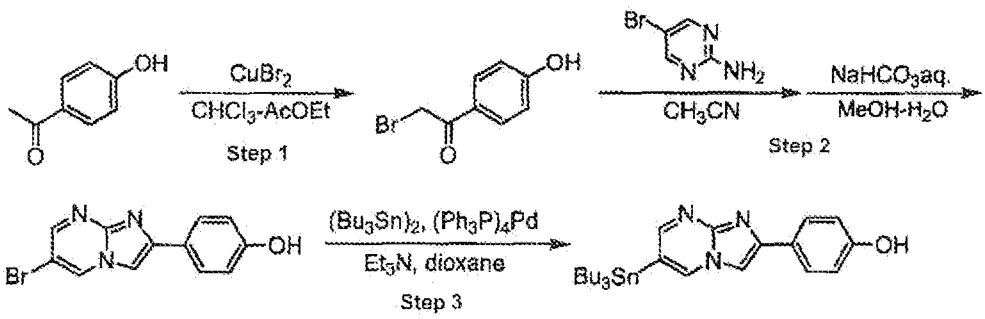


Figure 10

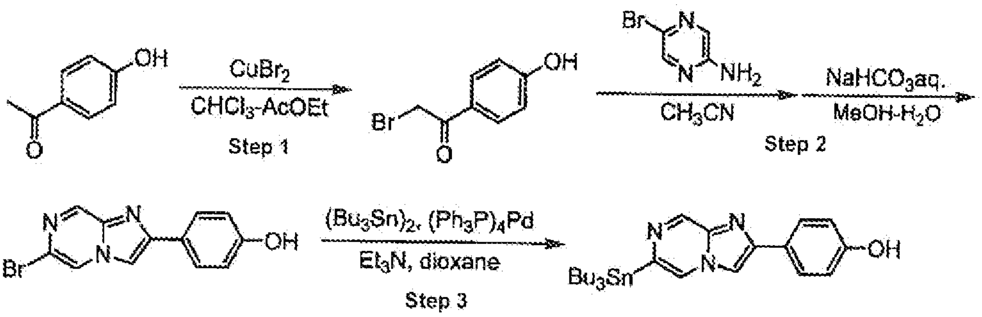


Figure 11

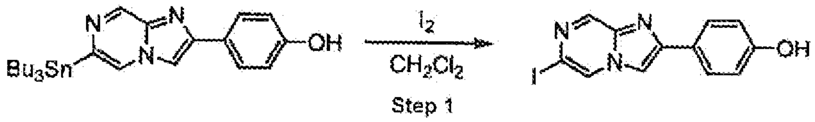


Figure 12

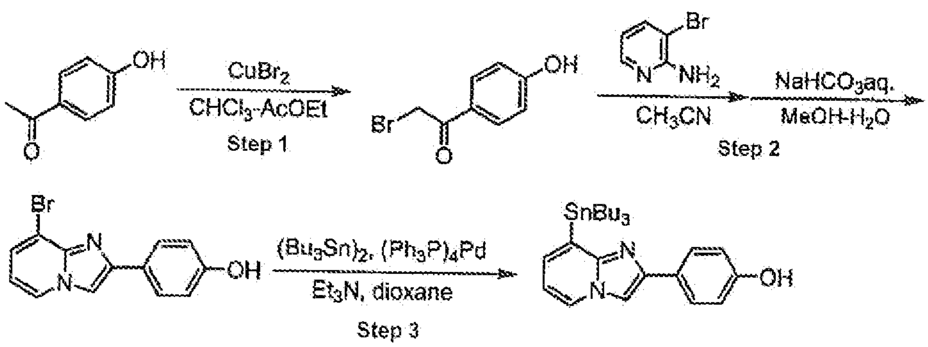


Figure 13

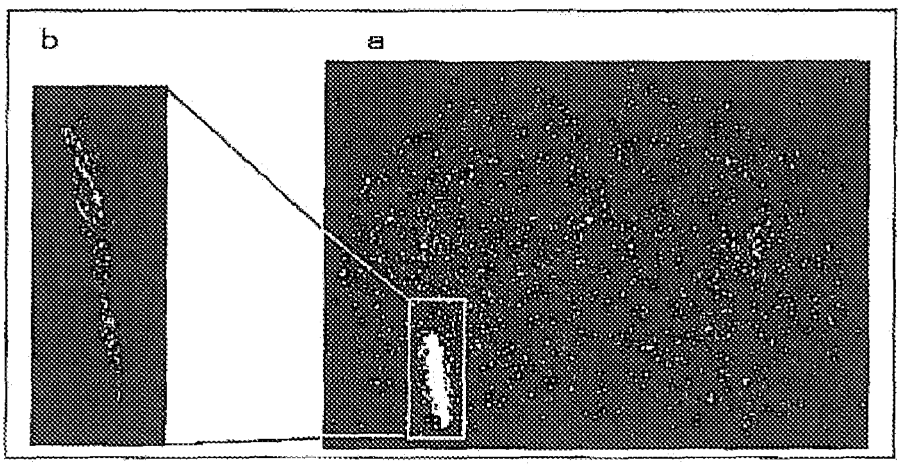


Figure 14

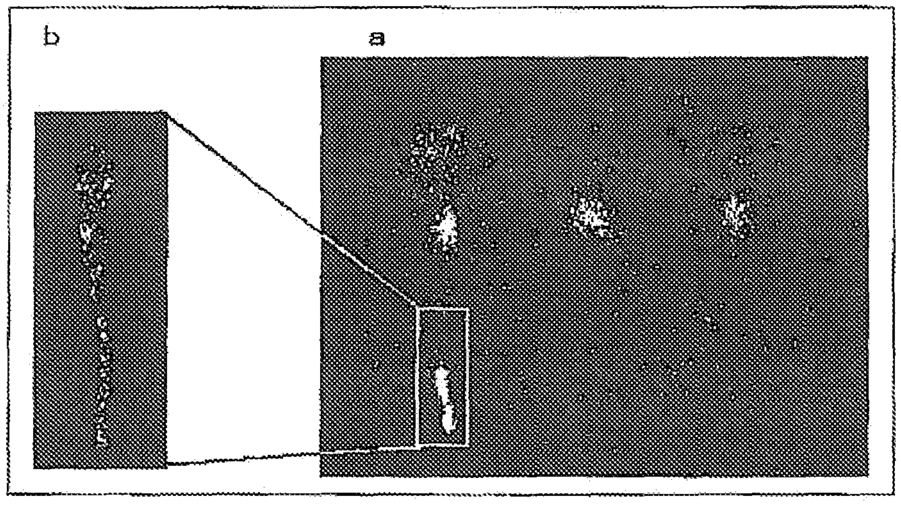


Figure 15

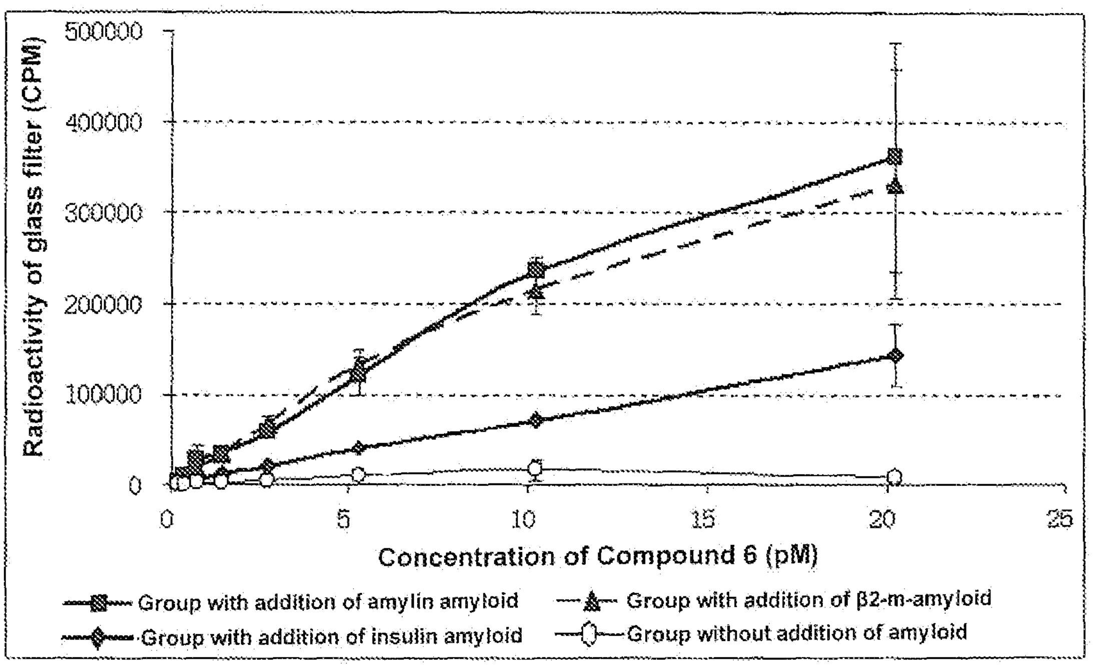
Radioactivity of glass filter (CPM)
500000
400000
300000
200000
100000
0
0
5
10
15
20
25
Concentration of Compound 6 (pM)
Group with addition of amylin amyloid
Group with addition of β2-m-amyloid
Group with addition of insulin amyloid
Group without addition of amyloid

Figure 16

**INTERNATIONAL SEARCH REPORT**

International application No.
PCT/JP2008/069501

A. CLASSIFICATION OF SUBJECT MATTER
*A61K51/00*(2006.01)i, *C07D471/04*(2006.01)i

According to International Patent Classification (IPC) or to both national classification and IPC

B. FIELDS SEARCHED

Minimum documentation searched (classification system followed by classification symbols)
A61K51/00, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), EMBASE(STN), MEDLINE(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| P,X | WO 2007/125988 A1 (Nihon Medi-Physics Co., Ltd.), 08 November, 2007 (08.11.07), Full text (Family: none) | 1-17 |
| A | JP 2005-512945 A (The Trustees of the University of Pennsylvania), 12 May, 2005 (12.05.05), & WO 2002/085903 A2 & EP 1381604 A2 & US 2004/0131545 A1 | 1-17 |
| A | JP 2002-523383 A (Regents of the University of California), 30 July, 2002 (30.07.02), & WO 2000/010614 A1 & EP 1105163 A1 & US 2002/0022002 A1 | 1-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 November, 2008 (21.11.08) | 02 December, 2008 (02.12.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/069501

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-506723 A (University of Pittsburgh), 04 March, 2004 (04.03.04), & WO 2002/016333 A2 & EP 1334091 A2 & US 2002/0133019 A1 | 1-17 |
| A | JP 2005-504055 A (The Trustees of the University of Pennsylvania), 10 February, 2005 (10.02.05), & WO 2003/018070 A1 & EP 1432453 A1 & US 2003/0149250 A1 | 1-17 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007002540 A
- WO 2007063946 A
- JP 2004506723 T
- JP 2005504055 T
- JP 2005512945 T
- JP 2002523383 T
- WO 2005016888 A
- WO 03106439 A


### Non-patent literature cited in the description


- **J. A. Hardy ; G. A. Higgins.** Alzheimer's Disease: The Amyloid Cascade Hypothesis. *Science,* 1992, vol. 256, 184-185
- **G. McKhann et al.** Clinical diagnosis of Alzheimer's disease: Report of the NINCDS-ADRDA Work Group under the auspices of Department of Health and Human Services Task Force on Alzheimer's Disease. *Neurology,* 1984, vol. 34, 939-944
- **Z.-P. Zhuang et al.** Radioiodinated Styrylbenzenes and Thioflavins as Probes for Amyloid Aggregate. *J. Med. Chem.,* 2001, vol. 44, 1905-1914
- **Masahiro Ono et al.** 11C-labeled stilbene derivatives as Aβ-aggregate-specific PET imaging agents for Alzheimer's disease. *Nuclear Medicine and Biology,* 2003, vol. 30, 565-571
- **H. F. Kung et al.** Novel Stilbenes as Probes for amyloid plaques. *J. American Chemical Society,* 2001, vol. 123, 12740-12741
- **Zhi-Ping Zhuang et al.** IBOX(2-(4'-dimethylaminophenyl)-6-iodobensoxazole): a ligand for imaging amyloid plaques in the brain. *Nuclear Medicine and Biology,* 2001, vol. 28, 887-894
- **Furumoto Y et al.** [11C]BF-227: A New C-Labeled 2-Ethenylbenzoxazole Derivative for Amyloid-β Plaques Imaging. *European Journal of Nuclear Medicine and Molecular Imaging,* 2005, vol. 32 (1), 759
- **Eric D. Agdeppa et al.** 2-Dialkylamino-6-Acylmalononitrile Substituted Naphthalenes (DDNP Analogs): Novel Diagnostic and Therapeutic Tools in Alzheimer's Disease. *Molecular Imaging and Biology,* 2003, vol. 5, 404-417
- **Zhi-Ping Zhuang et al.** Structure-Activity Relationship of Imidazo[1,2-a]pyridines as Ligands for Detecting β-Amyloid Plaques in the Brain. *J. Med. Chem,* 2003, vol. 46, 237-243
- **W. E. Klunk et al.** Imaging brain amyloid in Alzheimer's disease with Pittsburgh Compound-B. *Ann. Neurol.,* 2004, vol. 55, 306-319
- **Nicolaas P. L. G. Verhoeff et al.** In-Vivo Imaging of Alzheimer Disease β-Amyloid With [11C]SB-13 PET. *American Journal of Geriatric Psychiatry,* 2004, vol. 12, 584-595
- **Hiroyuki Arai et al.** [11C]-BF-227 AND PET to Visualize Amyloid in Alzheimer's Disease Patients. *Alzheimer's & Dementia: The Journal of the Alzheimer's Association,* 2006, vol. 2 (1), 312
- **Christopher M. Clark et al.** Imaging Amyloid with I123 IMPY SPECT. *Alzheimer's & Dementia: The Journal of the Alzheimer's Association,* 2006, vol. 2 (1), 342
- **D. M. Skovronsky et al.** *Proc. Natl. Acad. Sci.,* 2000, vol. 97, 7609
- **Zhi-Ping Zhuang et al.** *Nuclear Medicine and Biology,* 2001, vol. 28, 887-894
- **H. F. Kung et al.** *J. Am. Chem. Soc.,* 2001, vol. 123, 12740-12741
- **Masahiro Ono et al.** *Nuclear Medicine and Biology,* 2003, vol. 30, 565-571
- **king, L. Carroll ; Ostrum, G. Kenneth.** *Journal of Organic Chemistry,* 1964, vol. 29 (12), 3459-3461
- **Zhi-Ping Zhuang et al.** *J. Med. Chem,* 2003, vol. 46, 237-243
- **Naiki, H. et al.** *Laboratory Investigation,* 1996, vol. 74, 374-383
- **Wang, Y. et al.** *J. Labeled Compounds Radiopharmaceut.,* 2001, vol. 44, 239
- **Wang. Y. et al.** *J. Labelled Compounds Radiopharmaceut.,* 2001, vol. 44, 239
- **Zhuang, Z. P. et al.** *J. Med. Chem.,* 2003, vol. 46, 237
- **Douglas D. Dischino et al.** *J. Nucl. Med.,* 1983, vol. 24, 1030-1038
- **Burke, M.J. et al.** *Biochemistry.,* 1972, vol. 11, 2435-2439
- **Ohhashi, Y. et al.** *Journal of Biological Chemistry,* 2005, vol. 280, 32843-32848